(19) Europäisches Patentamt
European Patent Office
Office européen des brevets

(11) **EP 4 435 793 A2**

(12) **EUROPEAN PATENT APPLICATION**

(43) Date of publication:
**25.09.2024 Bulletin 2024/39**

(21) Application number: **24163283.5**

(22) Date of filing: **13.03.2024**

(51) International Patent Classification (IPC):
***G16C 20/20*** (2019.01)

(52) Cooperative Patent Classification (CPC):
**G16C 20/20;** G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.03.2023 JP 2023048104**

(71) Applicant: **Jeol Ltd.
Akishima-shi, Tokyo 196-8558 (JP)**

(72) Inventors:
• **KUBO, Ayumi**
**Tokyo, 196-8558 (JP)**
• **UBUKATA, Masaaki**
**Tokyo, 196-8558 (JP)**
• **NAGATOMO, Kenji**
**Tokyo, 196-8558 (JP)**

(74) Representative: **Boult Wade Tennant LLP
Salisbury Square House
8 Salisbury Square
London EC4Y 8AP (GB)**

(54) **SAMPLE ANALYZING APPARATUS AND METHOD OF CREATING PYROLYSIS PRODUCT LIBRARY**

(57)     A pyrolysis product library (24) formed from a plurality of groups of predicted mass spectra corresponding to a plurality of resin candidates is created using a prediction model (13). An information processing unit (16) generates a plurality of measured mass spectra corresponding to a plurality of compounds generated due to pyrolysis of a resin sample (22). Next, the information processing unit (16) searches through the pyrolysis product library (24) based on the plurality of measured mass spectra, to thereby judge one or a plurality of contained resins contained in the resin sample (22).

FIG. 1

**Description**

TECHNICAL FIELD

The present disclosure relates to a sample analyzing apparatus and to a method of creating a pyrolysis product library, and in particular to a technique for analyzing a sample through mass spectrometry of a pyrolysis product generated from the sample.

BACKGROUND

Various sample analyzing systems are known, including a sample analyzing system (Py-GC-MS system) which includes a pyrolysis apparatus, a gas chromatograph, and a mass spectrometry apparatus. Such a system will hereinafter be referred to as a pyrolysis analysis system.

In the pyrolysis analysis system, the sample to be analyzed is typically a polymer (in general, a resin). In the pyrolysis apparatus, a mixture gas including a plurality of pyrolysis products is generated from a sample introduced to the pyrolysis apparatus. The mixture gas is caused to pass through a column of the gas chromatograph, and, consequently, the plurality of pyrolysis products are separated. The plurality of pyrolysis products are a plurality of components generated from the sample, and are a plurality of compounds. The plurality of compounds are sequentially introduced to the mass spectrometry apparatus. In the mass spectrometry apparatus, mass spectrometry is performed for each compound. As a result, a mass spectrum array (which may also be called Py-GC-MS data) formed from a plurality of mass spectra arranged on a retention time axis is generated.

The pyrolysis analysis system described above generally has an information processing apparatus which identifies the sample based on the mass spectrum array. In the information processing apparatus of the related art, for example, a combined mass spectrum which represents the entirety of the mass spectrum array is generated from all or a primary portion of the mass spectrum array. Then, the combined mass spectrum is compared with a plurality of registered mass spectra. Based on the result of the comparison, the sample is identified (for example, refer to Document 1 (JP 2000-35422 A)).

In the case in which such a sample analyzing method is employed, if an impurity is mixed in the sample, a shape of the combined mass spectrum changes due to influences of the impurity, resulting in degradation of precision of analysis of the sample. In addition, such a sample analyzing method cannot be applied to a mixture sample formed from a plurality of samples. Further, when such a sample analyzing method is employed, if the pyrolysis condition or a component separation condition changes, the analysis of the sample becomes impossible.

Some existing compound databases register a mass spectrum of each compound (more specifically, an EI (Electron Ionization) mass spectrum). Such a database may be called a mass spectrum database. However, the existing mass spectrum database registers only a part of a large number of pyrolysis products (and a plurality of mass spectra corresponding thereto) generated due to pyrolysis of individual polymers. In the pyrolysis analysis, it is currently impossible to perform mass spectrum matching using the existing mass spectrum database as is.

Document 1 described above does not describe a sample analysis technique which uses artificially generated mass spectra.

An advantage of the present disclosure lies in realizing sample analysis of high reliability under the pyrolysis method. Alternatively, an advantage of the present disclosure lies in provision of a sample analyzing apparatus which can analyze the sample even when the pyrolysis condition or the component separation condition changes. Alternatively, an advantage of the present disclosure lies in provision of a pyrolysis product library which enables identification of a large number of pyrolysis products.

SUMMARY

According to one aspect of the present disclosure, there is provided a sample analyzing apparatus comprising: a measurement unit that performs mass spectrometry on a plurality of compounds generated due to pyrolysis of a polymer sample; a generation unit that generates a measured mass spectrum set, formed from a plurality of measured mass spectra corresponding to the plurality of compounds, based on data which is output from the measurement unit; and an analyzing unit that analyzes the polymer sample by comparing the measured mass spectrum set with all or a part of a plurality of groups of predicted mass spectra corresponding to a plurality of polymer candidates, wherein the group of predicted mass spectra corresponding to each of the polymer candidates includes a plurality of predicted mass spectra corresponding to a plurality of pyrolysis products which may be generated from the polymer candidate.

According to another aspect of the present disclosure, there is provided a program executed by an information processing apparatus, which, when executed, causes the information processing apparatus to realize the functions of: comparing a measured mass spectrum set, formed from a plurality of measured mass spectra corresponding to a plurality of compounds generated due to pyrolysis of a polymer sample, with all or a part of a plurality of groups of predicted mass spectra corresponding to a plurality of polymer candidates; and analyzing the polymer sample based on a result of comparison of the measured mass spectrum set with all or a part of the plurality of groups of predicted mass spectra, wherein the group of predicted mass spectra corresponding to each of the polymer candidates includes a plurality of predicted mass spectra corresponding to a plurality of pyrolysis products which may be generated from the polymer candidate.

According to another aspect of the present disclosure, there is provided a method of creating a pyrolysis product library, comprising: generating, for each polymer candidate of a plurality of polymer candidates, a plurality of structural formulae representing a plurality of pyrolysis products theoretically derived from the polymer candidate; generating, for each of the polymer candidates, a group of predicted mass spectra, formed from a plurality of predicted mass spectra, based on the plurality of structural formulae; and creating a pyrolysis product library including a plurality of groups of predicted mass spectra corresponding to the plurality of polymer candidates.

BRIEF DESCRIPTION OF DRAWINGS

Embodiment(s) of the present disclosure will be described based on the following figures, wherein:

FIG. 1 is a block diagram showing a sample analyzing system according to a first embodiment of the present disclosure;
FIG. 2 is a block diagram showing a prediction model generating apparatus;
FIG. 3 is a block diagram showing a library creating apparatus;
FIG. 4 is a block diagram showing a measurement unit;
FIG. 5 is a block diagram showing an information processing unit;
FIG. 6 is a diagram showing an example of a resin candidate list;
FIG. 7 is a block diagram showing a method of generating a group of compounds;
FIG. 8 is a diagram showing a first example of an original compound;
FIG. 9 is a diagram showing a second example of the original compound;
FIG. 10 is a diagram showing a first example of cutting of the original compound;
FIG. 11 is a diagram showing a second example of cutting of the original compound;
FIG. 12 is a diagram showing an example of bond changing;
FIG. 13 is a diagram showing an example of a pyrolysis product library;
FIG. 14 is a diagram showing a management table;
FIG. 15 is a flowchart showing a method of creating a pyrolysis product library;
FIG. 16 is a flowchart showing a method of analyzing a sample;
FIG. 17 is a block diagram showing a measurement unit according to a second embodiment of the present disclosure;
FIG. 18 is a diagram showing a method of analyzing a sample according to the second embodiment of the present disclosure;
FIG. 19 is a diagram showing a management table according to the second embodiment of the present disclosure;
FIG. 20 is a block diagram showing a sample analyzing system according to a third embodiment of the present disclosure;
FIG. 21 is a diagram showing a method of analyzing a sample according to the third embodiment of the present disclosure;
FIG. 22 is a diagram showing a management table according to the third embodiment of the present disclosure;
FIG. 23 is a diagram showing an example display in the third embodiment of the present disclosure; and
FIG. 24 is a diagram showing a method of analyzing a sample according to a fourth embodiment of the present disclosure.

DESCRIPTION OF EMBODIMENTS

Embodiments of the present disclosure will now be described with reference to the drawings.

(1) Overview of Embodiments

A sample analyzing apparatus according to an embodiment of the present disclosure comprises a measurement unit, a generation unit (generator), and an analyzing unit (analyzer). The measurement unit has a mass spectrometer. The measurement unit performs mass spectrometry on a plurality of compounds generated due to pyrolysis of a polymer sample. The generation unit generates a measured mass spectrum set, formed from a plurality of measured mass spectra corresponding to the plurality of compounds, based on data which is output from the measurement unit. The analyzing unit analyzes the polymer sample by comparing the measured mass spectrum set with all or a part of a plurality of groups of predicted mass spectra corresponding to a plurality of polymer candidates. The group of predicted mass spectra corresponding to each of the polymer candidates includes a plurality of predicted mass spectra corresponding to a plurality of pyrolysis products which may be generated from the polymer candidate.

The above-described structure analyzes the polymer sample through individual identification of a plurality of compounds generated due to pyrolysis of the polymer sample (that is, a plurality of pyrolysis products). Therefore, according to the structure described above, even when an impurity is contained in the polymer sample, the polymer sample can be analyzed without being significantly affected by the impurity. In addition, according to the structure described above, because a result of mass spectrum comparison can be obtained for each group of predicted mass spectra (that is, for each polymer candidate), it becomes possible to simultaneously identify a plurality of polymers contained in the polymer sample. Moreover, according to the structure described above, the polymer can be analyzed even when the pyrolysis condition or the component separation condition changes.

In the measurement unit according to the embodiment of the present disclosure, pyrolysis, component separation, and mass spectrometry are sequentially performed. In the embodiment, each measured mass spectrum is an accumulated mass spectrum corresponding to a compound peak to be described later. Alternatively, as each of the measured mass spectra, other mass spectra corresponding to the compound peak may be employed.

In an embodiment of the present disclosure, each of the predicted mass spectra is an artificial mass spectrum generated based on each pyrolysis product theoretically derived from the polymer candidate. Specifically, each of the predicted mass spectra is a mass spectrum generated by supplying a structural formula representing each of the pyrolysis products to a trained prediction model.

In general, it is very difficult to identify or extract a plurality of mass spectra corresponding to a plurality of pyrolysis products from a mass spectrum array obtained through pyrolysis of the polymer. On the other hand, a plurality of pyrolysis products which may be generated from the polymer can be theoretically (and comprehensively) predicted. Further, a mass spectrum can also be predicted from the structural formula of each of the predicted pyrolysis products. From such a viewpoint, in the embodiment of the present disclosure, a plurality of groups of predicted mass spectra corresponding to a plurality of polymer candidates are generated in advance, and the polymer sample is analyzed using these groups of predicted mass spectra. Alternatively, individual predicted mass spectra may be generated at the time when the mass spectrum matching is performed.

In an embodiment of the present disclosure, the analyzing unit calculates a score for each of the polymer candidates by performing mass spectrum matching between the measured mass spectrum set and all or a part of the plurality of groups of predicted mass spectra. The analyzing unit then judges one or a plurality of polymers contained in the polymer sample based on the score for each of the polymer candidates.

For example, when the polymer sample contains a particular polymer candidate, the mass spectrum matching between the measured mass spectrum set and the group of predicted mass spectra corresponding to the particular polymer candidate yields superior result, and results in a high score. On the other hand, when the polymer sample does not contain the particular polymer candidate, the mass spectrum matching between the measured mass spectrum set and the group of predicted mass spectra corresponding to the particular polymer candidate yields inferior result, and results in a low score. Thus, the probability of containing each polymer candidate can be judged based on the respective score. The score is an index indicating a degree of spectrum matching.

In an embodiment of the present disclosure, the analyzing unit calculates a similarly matrix for each of the polymer candidates as a result of the mass spectrum matching. The analyzing unit then calculates the score based on the similarly matrix, for each of the polymer candidates. In an embodiment of the present disclosure, the analyzing unit counts a number of similarities which are higher than a threshold in the similarly matrix, for each of the polymer candidates. The analyzing unit calculates the score based on the number of similarities which are higher than the threshold, for each of the polymer candidates.

For example, when the measured mass spectrum set is formed from i measured mass spectra, and j predicted mass spectra predicted from j pyrolysis products are correlated to a certain polymer candidate, (i x j) similarities are calculated, and a similarity matrix is formed therefrom. In this case, if the polymer sample contains the polymer candidate, a relatively large number of high similarities are caused. As the number of the high similarities becomes larger, the probability that the polymer sample contains the polymer candidate becomes higher. In general, j differs depending on the polymer candidate. When the score is to be calculated for each of the polymer candidates, the actual value of j may or may not be taken into consideration. By performing screening prior to the mass spectrum matching, the number of predicted mass spectra to be compared with each of the measured mass spectra can be reduced. Through the screening, a portion of the plurality of polymer candidates may be removed from identification targets.

In an embodiment of the present disclosure, the analyzing unit extracts a selected predicted mass spectrum array to be compared with each of the measured mass spectra, from among the plurality of groups of predicted mass spectra, based on molecular mass information of each of the compounds. According to this structure, the screening of the pyrolysis products (or polymer candidates) can be performed based on the molecular mass information.

In an embodiment of the present disclosure, the molecular mass information is an accurate mass of the molecule of each compound generated due to pyrolysis. Alternatively, information other than the accurate mass may be employed as the molecular mass information.

In an embodiment of the present disclosure, the analyzing unit estimates, based on the molecular mass information of each of the compounds, a structural formula of the compound. The analyzing unit extracts the selected predicted mass spectrum array by comparing the structural formula which is estimated with a group of structural formulae corresponding to a group of pyrolysis products of each of the polymer candidates. According to this structure, analysis precision of the polymer sample can be improved through combinational use (more specifically, a stepwise application) of the structural formula matching and the mass spectrum matching.

In an embodiment of the present disclosure, the measurement unit has a first ion source and a second ion source which differ from each other. The generation unit generates a first measured mass spectrum set based on first data which is output from the measurement unit when the first ion source is used. In addition, the generation unit generates a second measured mass spectrum set based on second data which is output from the measurement unit when the second ion source is used. The analyzing unit identifies a plurality of molecular mass information of the plurality of compounds based on a plurality of molecular ion peaks included in the second measured mass spectrum set.

For example, the first ion source is an ion source which follows a hard ionization method, and the second ion source is an ion source which follows a soft ionization method. The hard ionization method is an ionization method in which, in comparison to the soft ionization method, fragment ions tend to be more easily produced. The soft ionization method is an ionization method in which, in comparison to the hard ionization method, molecular ions tend to be more easily produced.

The sample analyzing apparatus according to an embodiment of the present disclosure further comprises: a pyrolysis product library having the plurality of groups of predicted mass spectra; and a compound library having a mass spectrum set corresponding to a known compound set. The analyzing unit searches through the pyrolysis product library and the compound library based on the measured mass spectrum set. The analyzing unit judges, for each of the measured mass spectra, an attribute of a compound corresponding to the measured mass spectrum, based on result of search of the pyrolysis product library and the compound library. According to this structure, precision of identification of the attribute of the compound can be improved through the combinational use of two libraries. For example, as the attribute of the compound, a first attribute which indicates that the compound is a pyrolysis product of a polymer candidate, or a second attribute which indicates that the compound is not a pyrolysis product of the polymer candidate (that is, the compound is another compound) is judged.

In an embodiment of the present disclosure, the mass spectrum set includes a plurality of predicted mass spectra generated from a plurality of known structural formulae corresponding to a plurality of known compounds. For example, the mass spectrum set is a predicted mass spectrum set. According to this structure, a compound library can be easily created based on a known compound database.

In an embodiment of the present disclosure, the analyzing unit identifies the attribute of the compound by identifying a library to which a mass spectrum resulting in a highest similarity belongs. That is, when the mass spectrum which results in the maximum similarity belongs to the pyrolysis product library, the first attribute is judged as the attribute of the compound. On the other hand, when the mass spectrum which results in the maximum similarity belongs to the compound database, the second attribute is judged as the attribute of the compound.

The sample analyzing apparatus according to an embodiment of the present disclosure further comprises: a TICC generator and a reference image generator. The TICC generator generates a total ion current chromatogram (TICC) including a plurality of compound peaks corresponding to a plurality of measured mass spectra, based on the data which is output from the measurement unit. The reference image generator generates a reference image to be displayed along with the TICC, based on attributes of a plurality of compounds identified according to the plurality of measured mass spectra. The reference image includes a plurality of markers to be displayed along with the plurality of compound peaks, which show the attributes of the plurality of compounds. According to this structure, it is possible to easily judge whether or not the compound peak included in the TICC is a peak derived from a pyrolysis product, through observation of the reference image.

According to another aspect of the present disclosure, there is provided a program which is executed by an information processing apparatus, which, when executed, causes the information processing apparatus to realize a first function and a second function.

The first function is a function to compare a measured mass spectrum set, formed from a plurality of measured mass spectra corresponding to a plurality of compounds generated due to pyrolysis of a polymer sample, with all or a part of a plurality of groups of predicted mass spectra corresponding to a plurality of polymer candidates. The second function is a function to analyze the polymer sample based on a result of comparison of the measured mass spectrum set with all or a part of the plurality of groups of predicted mass spectra. The group of predicted mass spectra corresponding to each of the polymer candidates includes a plurality of predicted mass spectra corresponding to a plurality of pyrolysis products which may be generated from the polymer candidate.

The information processing apparatus is, for example, a computer, a mass spectrometry apparatus, or a sample analyzing system. The program is installed to the information processing apparatus via a network or a transportable recording medium. The information processing apparatus has a non-transitory recording medium which stores the program.

According to another aspect of the present disclosure, there is provided a method of creating a pyrolysis product library, comprising a first step, a second step, and a third step. In the first step, for each polymer candidate of a plurality of polymer candidates, a plurality of structural formulae representing a plurality of pyrolysis products theoretically derived from the polymer candidate are generated. In the second step, for each of the polymer candidates, a group of predicted mass spectra, formed from a plurality of predicted mass spectra, is generated based on the plurality of structural formulae. In the third step, a pyrolysis product library including a plurality of groups of predicted mass spectra corresponding to the plurality of polymer candidates is created.

In the creating method described above, a plurality of pyrolysis products are theoretically generated, and a plurality of predicted mass spectra which are artificial are generated from the pyrolysis products. According to the creating method described above, a pyrolysis product library, which is difficult to create through experiments, can be artificially created.

In an embodiment of the present disclosure, the step of generating the plurality of structural formulae comprises a step of generating an original structural formula of an original pyrolysis product serving as a monomer connecting structure, and a step of generating a plurality of derived structural formulae from the original structural formula. The plurality of structural formulae include the original structural formula and the plurality of derived structural formulae. A polymer has a polymerization degree distribution of, for example, a few hundreds to a few tens of thousands. Through pyrolysis of such a polymer, various pyrolysis products which are relatively small are generated. The pyrolysis products include a pyrolysis product formed from k repeating units. Here, k is typically 1, 2, 3, 4, or 5. Based on this knowledge, desirably, for example, an original pyrolysis product formed from 6 or more repeating units is generated, and a plurality of derived pyrolysis products are created from the original pyrolysis product.

(2) Details of Embodiments

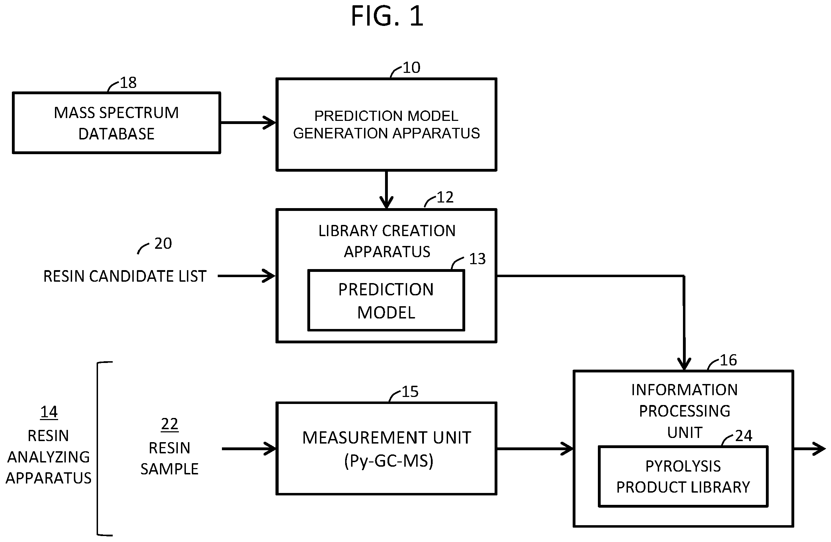

FIG. 1 shows a sample analyzing system according to a first embodiment of the present disclosure. The sample analyzing system is specifically a polymer analyzing system, and more specifically a resin analyzing system. The resin analyzing system is a system for identifying one or a plurality of resins contained in a resin sample 22. Alternatively a sample other than the resin may be analyzed.

The resin analyzing system comprises a prediction model generation apparatus 10, a library creation apparatus 12, and a resin analyzing apparatus 14. The prediction model generation apparatus 10 and the library creation apparatus 12 function prior to the actual analysis of the sample, and the apparatus that functions during the actual sample analysis is the resin analyzing apparatus 14.

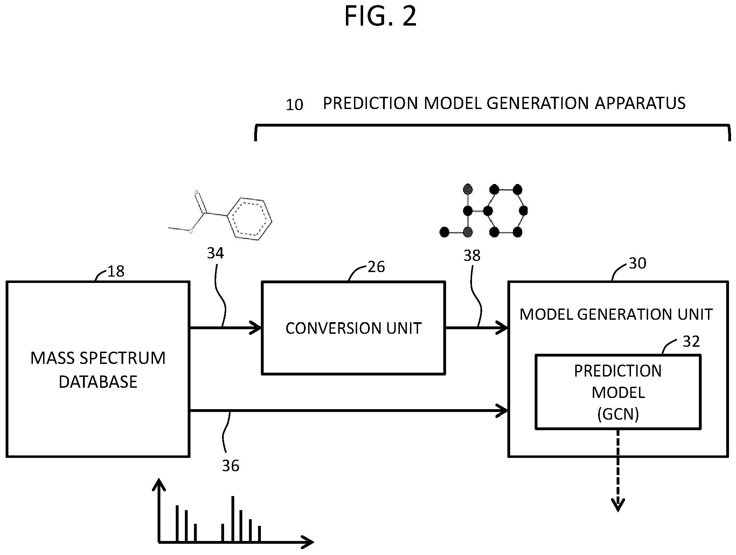

The prediction model generation apparatus 10 is an apparatus which generates a prediction model through machine learning based on information stored in an existing mass spectrum database 18. In the mass spectrum database 18, for example, various compounds and various EI mass spectra corresponding thereto are registered. As a specific example of the mass spectrum database, the NIST (National Institute of Standards and Technology) Mass Spectral Library may be exemplified. The generated prediction model is transferred to the library creation apparatus 12. An example structure of the prediction model generation apparatus 10 will be described later with reference to FIG. 2.

Prior to the analysis of the resin sample, a resin candidate list 20 formed from a plurality of resin candidates is created in advance. The actual substance of each resin candidate is a resin identifier such as the resin name. The plurality of resin candidates are designated, for example, by a user. The number of the resin candidates is, for example, a few tens. A larger number of resin candidates or a smaller number of resin candidates may be designated. Each resin candidate is a resin which may be contained in the resin sample 22, and is a candidate for identification.

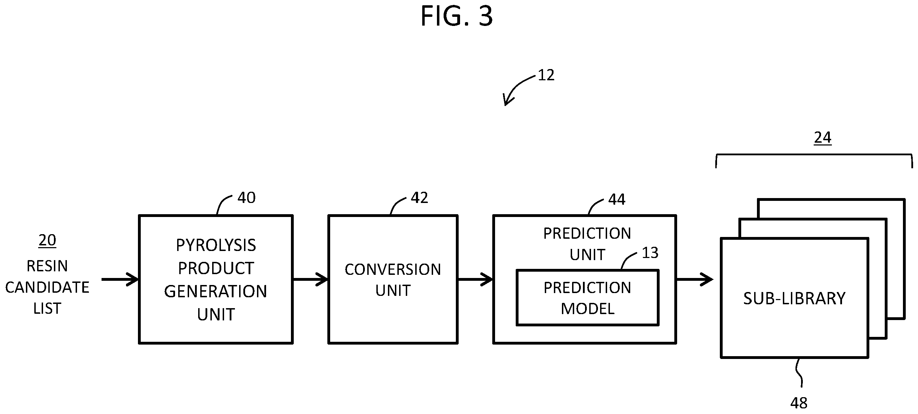

The library creation apparatus 12 is an apparatus which creates a pyrolysis product library using a prediction model 13 generated by the prediction model generation apparatus 10. The library creation apparatus 12 creates a group of pyrolysis products (more specifically, a group of structural formulae) for each resin candidate in the resin candidate list 20, and then, supplies the group of pyrolysis products to the prediction model 13, to generate a group of predicted mass spectra. By repeating these processes, a plurality of groups of predicted mass spectra corresponding to the plurality of resin candidates are generated. The pyrolysis product library is formed by the plurality of groups of predicted mass spectra. The pyrolysis product library is installed in the resin analyzing apparatus 14. An example structure of the library creation apparatus 12 will be described later with reference to FIG. 3.

The resin analyzing apparatus 14 has a measurement unit 15 and an information processing unit 16. The measurement unit 15 has a pyrolysis apparatus, a gas chromatograph, and a mass spectrometer. In the pyrolysis apparatus, through pyrolysis of the resin sample 22 which is the polymer sample, a mixture gas formed from a plurality of pyrolysis products is generated. The mixture gas is introduced to the gas chromatograph, and the plurality of pyrolysis products are separated. The plurality of pyrolysis products which are separated are a plurality of components generated from the resin sample 22, and are a plurality of compounds. The plurality of compounds are sequentially introduced to the mass spectrometer. In the mass spectrometer, mass spectrometry is performed on the plurality of compounds.

In the information processing unit 16, a plurality of mass spectra which are arranged on a retention time axis are generated based on the data which is output from the measurement unit 15. A mass spectrum array is formed from the plurality of mass spectra. Then, for each pyrolysis product; that is, for each compound, a plurality of mass spectra are accumulated, and an accumulated mass spectrum is generated. With this process, a plurality of accumulated mass spectra are generated corresponding to the plurality of compounds. Each accumulated mass spectrum is handled as a measured mass spectrum for identifying the compound.

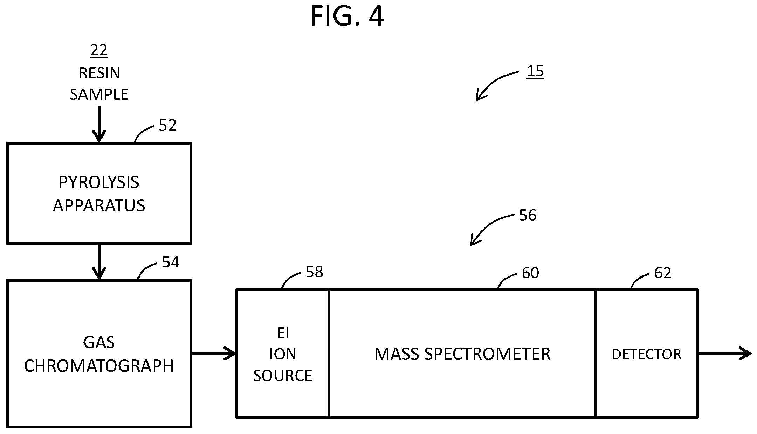

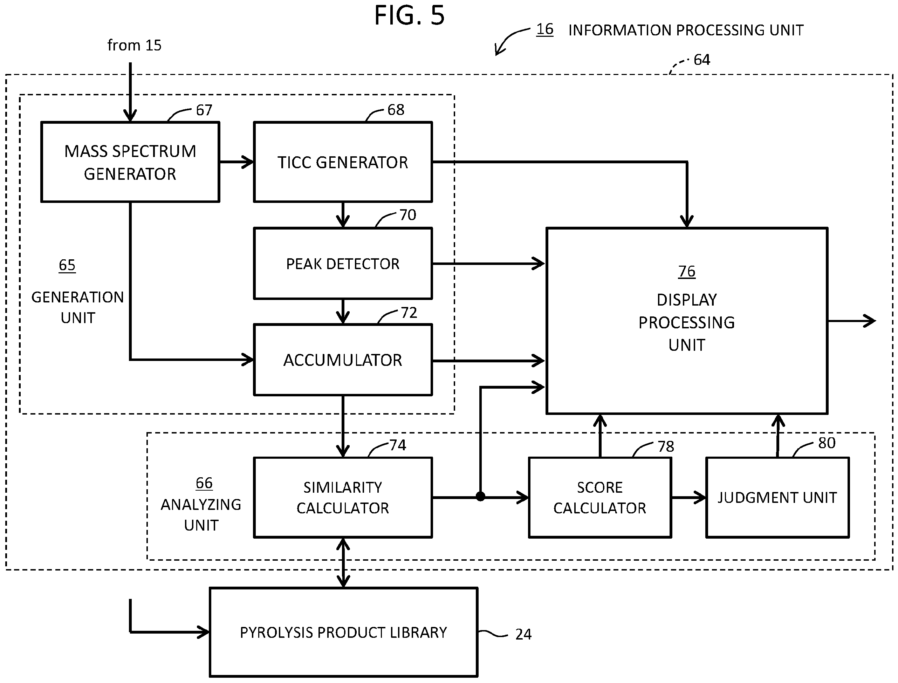

The information processing unit 16 searches through a pyrolysis product library 24 based on the plurality of measured mass spectra corresponding to the plurality of compounds, to thereby identify one or a plurality of contained resins contained in the resin sample 22. An example of the specific structure of the measurement unit 15 will be described later with reference to FIG. 4. An example of the specific structure of the information processing unit 16 will be described later with reference to FIG. 5.

In the embodiment, each of the prediction model generation apparatus 10, the library creation apparatus 12, and the information processing unit 16 is formed from a computer. The prediction model generation apparatus 10 and the library creation apparatus 12 may be formed from a single computer. Alternatively, the prediction model generation apparatus 10, the library creation apparatus 12, and the information processing unit 16 may be formed from a single computer. A part or all of a sequence of functions realized by the prediction model generation apparatus 10, the library creation apparatus 12, and the information processing unit 16 may be executed by one or a plurality of computers on a network.

An example structure of the prediction model generation apparatus 10 will now be described with reference to FIG. 2. In an example structure illustrated in FIG. 2, the prediction model generation apparatus 10 has a conversion unit 26 and a model generation unit 30. The conversion unit 26 is a module which generates graph structure data from the structural formula of the compound. The model generation unit 30 functions as a learner. More specifically, in the embodiment, the model generation unit 30 is formed from GCN (Graph Convolutional Networks). That is, the GCN is the actual substance of a prediction model 32. Alternatively, other networks or other models may be employed in place of the GCN.

In the mass spectrum database 18, a large volume of compound information is registered. Specifically, a large number of structural formulae and a large number of mass spectra corresponding to the large number of the compounds are registered. The large number of structural formulae and the large number of mass spectra as a whole are used as a training data set for machine learning. Individual training data is formed from a structural formula 34 and a mass spectrum 36 corresponding thereto. The structural formula 34 is converted to graph structure data 38 at the conversion unit 26. The mass spectrum 36 is used as the ground truth data. From the viewpoint of the GCN, a pair consisting of the graph structure data 38 and the mass spectrum 36 is the actual training data.

A large number of training data are supplied to the model generation unit 30. With this process, the predicted mass spectrum which is output from the prediction model 32 is gradually improved. Through such a machine leaning process, the prediction model 32 for predicting the mass spectrum from an arbitrary structural formula is completed.

FIG. 3 shows an example structure of the library creation apparatus 12. In the example structure illustrated in FIG. 3, the library creation apparatus 12 has a pyrolysis product generation unit 40, a conversion unit 42, and a prediction unit 44. The pyrolysis product generation unit 40 comprehensively generates, for each resin candidate in the resin candidate list 20, a large number of pyrolysis products. Specifically, the pyrolysis product generation unit 40 generates a large number of structural formulae representing the large number of pyrolysis products, for each resin candidate. The structural formulae form a group of structural formulae.

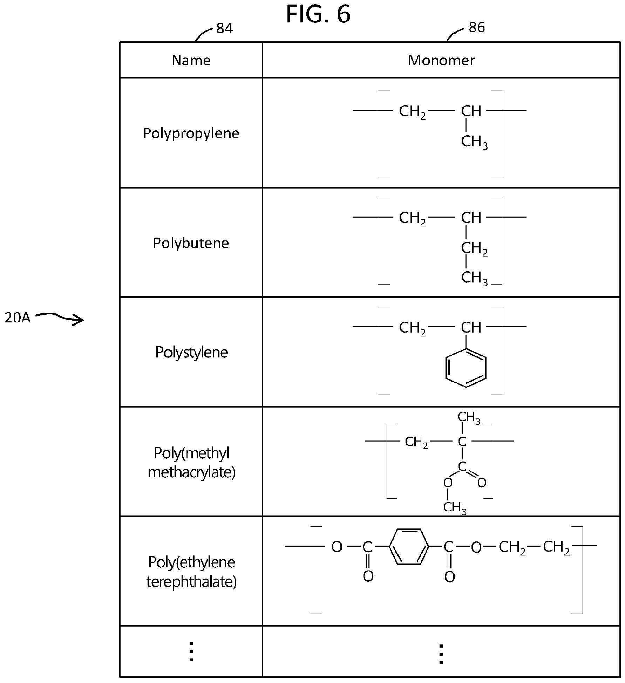

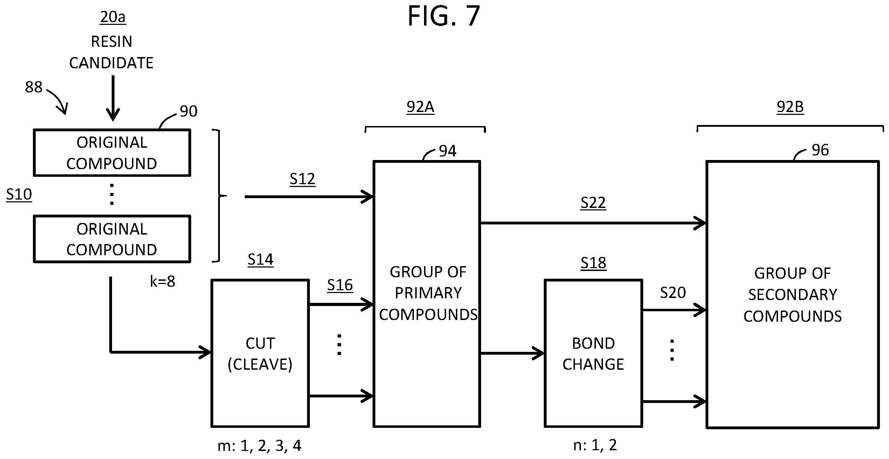

Various methods are known as a notation for the structural formula. For example, there are known a MOL SDF file format, linear notations such as SMILES and SMARTS, and the like. An example of the resin candidate list 20 will be described later with reference to FIG. 6. A method of generating the group of pyrolysis products (group of structural formulae) will be described later in detail with reference to FIGs. 7 to 12.

The conversion unit 42 converts the structural formula which is input into the graph structure data. The converted graph structure data is supplied to the prediction model 13 in the prediction unit 44. With this process, a predicted mass spectrum is generated for each structural formula; that is, for each pyrolysis product. One group of predicted mass spectra is generated for one resin candidate. One sub-library 48 is formed by one group of predicted mass spectra. The pyrolysis product library 24 is formed from a plurality of sub-libraries 48 corresponding to a plurality of resin candidates. Alternatively, in the conversion unit 42, the structural formula which is input may be converted to a data format adapted to the model other than the graph structure, such as, for example, a finger print.

The pyrolysis product library 24 is a virtual library which is not generated through experiments, and is artificially generated. It is very difficult to construct a sub-library through analysis of a mass spectrum array obtained through mass spectrometry of a plurality of compounds generated due to pyrolysis of the resin sample. On the contrary, according to the present embodiment, such a sub-library can be easily created.

FIG. 4 shows an example structure of the measurement unit 15 in the resin analyzing apparatus. As described above, the measurement unit 15 includes a pyrolysis apparatus 52, a gas chromatograph 54, and a mass spectrometer 56. The mass spectrometer 56 has an EI ion source 58, a mass analyzer 60, and a detector 62.

In the pyrolysis apparatus 52, a mixture gas formed from a plurality of pyrolysis products is generated through pyrolysis of the resin sample 22. The mixture gas is introduced to a column in the gas chromatograph 54. With this process, a plurality of pyrolysis products (that is, a plurality of compounds) are separated timewise. These compounds are sequentially introduced to the mass spectrometer 56.

In the mass spectrometer 56, the EI ion source 58 is an ion source which follows the electron ionization method (EI method). The EI method is one of the hard ionization methods. When the sample is ionized through the EI method, fragment ions tend to be easily produced. The mass analyzer 60 is, for example, a quadrupole type mass analyzer, or a time-of-flight type mass analyzer. The ions passing through the mass analyzer 60 are detected by the detector 62. With this process, detection data (a detection signal) is output from the mass spectrometer 56.

FIG. 5 shows an example structure of the information processing unit 16 in the resin analyzing apparatus. The information processing unit 16 has a processor 64. The processor 64 is, for example, a CPU which executes a program. The information processing unit 16 has a memory. In the memory, the program is stored, and, in addition, the pyrolysis product library 24 is stored. Alternatively, a pyrolysis product library stored in a server on a network may be utilized. In the embodiment, a management table is stored in the memory, along with or including the pyrolysis product library. The management table is an operation table in which various information necessary during execution of the sample analysis and various information generated during the sample analysis are registered.

In FIG. 5, a plurality of functions realized by the processor 64 are represented with a plurality of blocks. A generation unit (generator) 65 has a mass spectrum generator 67, a TICC (Total Ion Current Chromatogram) generator 68, a peak detector 70, and an accumulator 72. The analyzing unit (analyzer) 66 has a similarity calculator 74, a score calculator 78, and a judgment unit 80. The processor 64 also functions as a display processing unit 76.

The detection data which is output from the measurement unit 15 is input to the mass spectrum generator 67. The mass spectrum generator 67 generate a plurality of mass spectra arranged along the retention time axis, based on the detection data which is input. A mass spectrum array is formed from the plurality of mass spectra.

The TICC generator 68 generates a total ion current chromatogram (TICC) based on the mass spectrum array. Specifically, for each mass spectrum, the mass spectra are accumulated, and a total ion current (TIC) is calculated. By plotting a change with respect to time of the TIC on the retention time axis, the TICC is generated. The TICC is also called a pyrogram.

The peak detector 70 is a module which detects a plurality of compound peaks included in the TICC. The plurality of compound peaks are basically a plurality of pyrolysis product peaks.

The accumulator 72 sets, for each compound peak, an accumulation segment on the retention time axis, and accumulates a plurality of mass spectra in the accumulation segment. With this process, an accumulated mass spectrum is generated for each compound peak. In the embodiment, the accumulated mass spectrum is handled as a measured mass spectrum corresponding to a particular compound (that is, a particular pyrolysis product). A plurality of measured mass spectra are generated based on the plurality of compound peaks. A measured mass spectrum set is formed by the plurality of the measured mass spectra.

The similarity calculator 74 accesses the pyrolysis product library 24 and refers to the plurality of groups of predicted mass spectra corresponding to the plurality of resin candidates. The similarity calculator 74 performs mass spectrum matching between the measured mass spectrum set and the plurality of groups of predicted mass spectra. With this process, a plurality of similarity matrices to be described later are determined.

For example, the similarity is calculated through the following equation.

[Equation 1]

$$1000 \frac{\sum_i m_i \sqrt{A_i \cdot P_i}}{\sqrt{\sum_i m_i \cdot A_i} \cdot \sqrt{\sum_i m_i \cdot P_i}}$$

In the equation described above, $m_i$ represents an ith mass, $A_i$ represents an ith intensity in the measured mass spectrum, and $P_i$ represent an ith intensity in the predicted mass spectrum. The equation described above is an equation for determining a cosine similarity. Alternatively, other similarities may be calculated.

The score calculator 78 calculates a plurality of scores based on the plurality of similarity matrices corresponding to the plurality of resin candidates. The score is an index showing a magnitude of probability that the resin sample contains the resin candidate. Prior to the calculation of the score, each similarity is compared to a threshold th. Specifically, it is judged whether or not each similarity is higher than the threshold th. Presuming the equation described above, the threshold th is, for example, 700. In the embodiment, a number of similarities exceeding the threshold th is set as the score. Alternatively, the score may be calculated through other methods. The threshold th may be defined for each resin candidate, or a common threshold th over a plurality of resin candidates may be defined.

The judgment unit 80 judges one or a plurality of candidate resins contained in the resin sample based on the plurality of scores corresponding to the plurality of resin candidates. For example, when the score exceeds a judgment value, the resin candidate corresponding to the score is judged as a contained resin. The judgment value is, for example, 10. The judgment value may be defined for each resin candidate, or a common judgment value over a plurality of resin candidates may be defined. Alternatively, a resin candidate corresponding to the highest score may be judged as the contained resin.

The display processing unit 76 generates an image to be displayed on a display. In the embodiment, the TICC is displayed on the display, and the result of judgment by the judgment unit 80 is displayed. Further, one or a plurality of measured mass spectra may be displayed on the display, or a score array may be displayed along with the resin candidate list.

FIG. 6 shows an example of the resin candidate list. A resin candidate list 20A illustrated in FIG. 6 has a resin name array 84 formed from a plurality of resin names, and a monomer structural formula array 86 formed from a plurality of monomer structural formulae.

FIG. 7 exemplifies a method of generating the group of pyrolysis products. An actual substance of each individual pyrolysis product; that is, each individual compound, is a structural formula. Based on a selected resin candidate 20a, an original compound array (original pyrolysis product array) 88 is generated (S10). The original compound array 88 is formed from one or a plurality of original compounds 90. Each individual original compound 90 is, for example, a connecting structure formed from k monomers. Here, k is, for example, an integer greater than or equal to 5, is desirably an integer in a range of 6 to 10, and is 8 in the embodiment. Alternatively, a numerical value of greater than or equal to 9 or smaller than or equal to 7 may be designated by the user as a value for k.

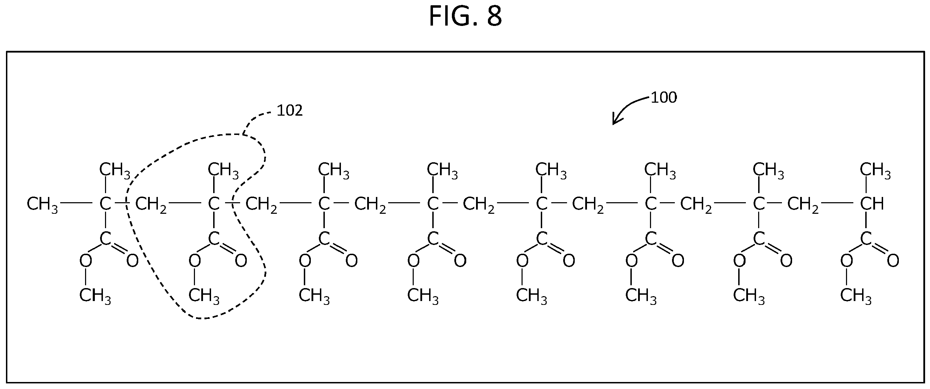

FIG. 8 shows an example of the original compound. An original compound 100 is formed from 8 monomers (repeating units) 102. In the example illustrated in FIG. 8, each end group is H.

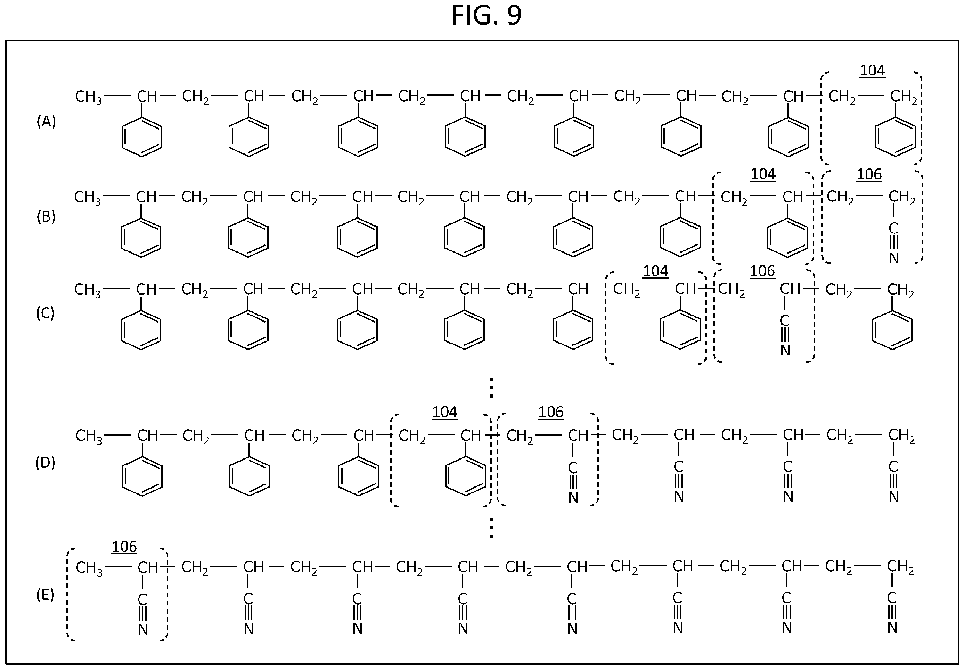

When the resin candidate is a copolymer, depending on a bonding form thereof (for example, random or block), for example, a series of original compounds as shown in FIG. 9 are generated. FIG. 9 shows in (A) an original compound formed from 8 first monomers 104, in (B) an original compound formed from 7 first monomers 104 and 1 second monomer 106, in (C) an original compound formed from 6 first monomers 104 and 2 second monomers 106, in (D) an original compound formed from 4 first monomers 104 and 4 second monomers 106, and in (E) an original compound formed from 8 second monomers 106. As described, for each resin candidate, all theoretically possible original compounds are generated according to the condition of k=8.

Referring to FIG. 7, a plurality of original compounds generated based on the plurality of resin candidates are registered in a memory 92A (S12). In the meantime, a cutting process is applied to each original compound generated in S10 (S14). The cutting process is an artificial manipulation simulating cleaving during pyrolysis. More specifically, in the embodiment, presuming k=8, numbers of 1, 2, 3, and 4 are sequentially selected as a number of cutting m, and cutting is repeated for each individual original compound as follows. With this process, a plurality of derived compounds are generated from one original compound.

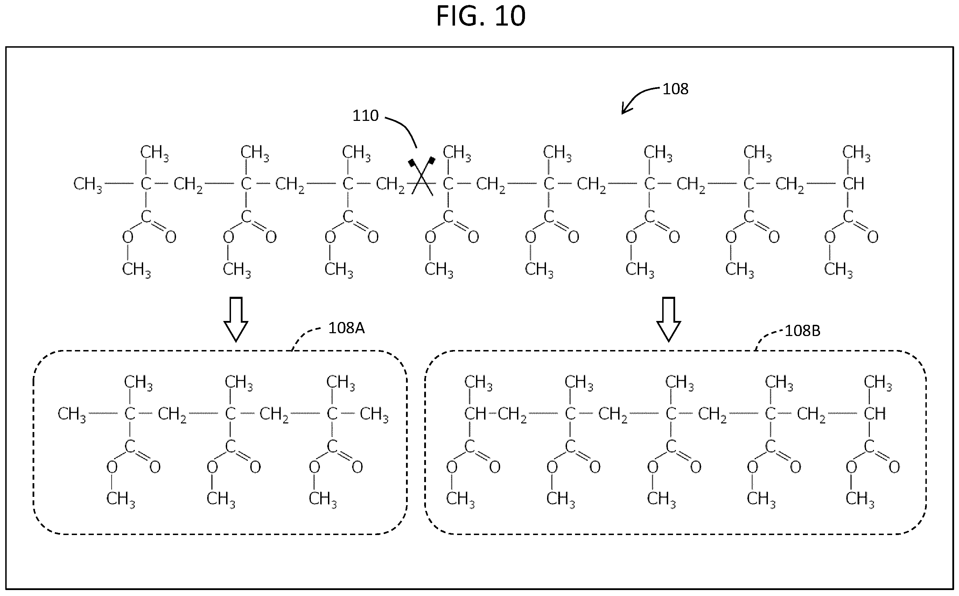

FIG. 10 shows an example of cutting when m=1. In an original compound 108, a designated location 110 is cut. With this process, two compounds 108A and 108B are generated from the original compound 108. While all locations which can be cut in the original compound 108 are sequentially selected as the designated location 110, the cutting of the original compound 108 is repeated.

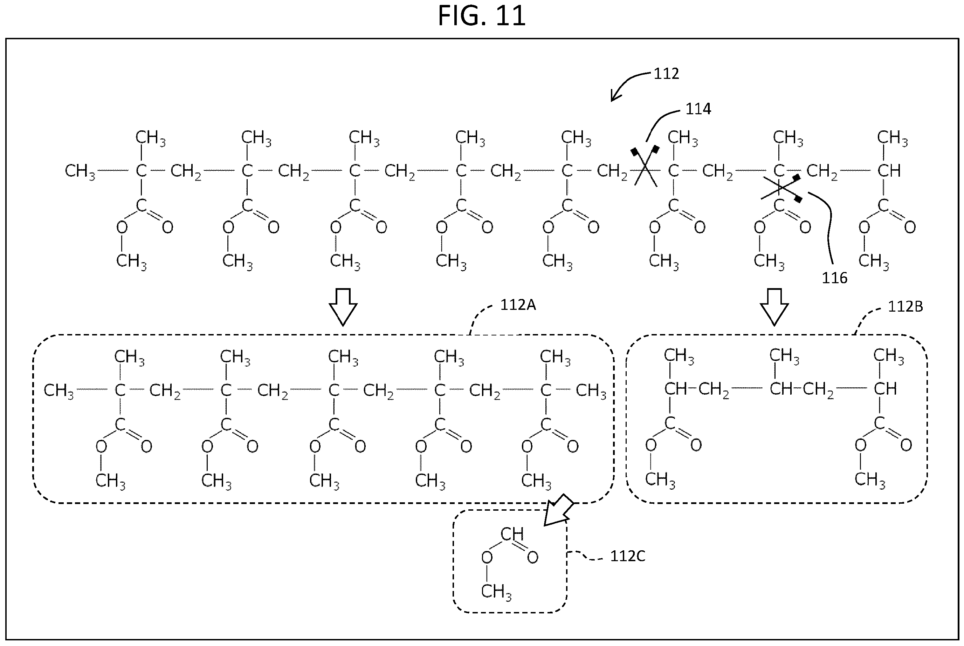

FIG. 11 shows an example of cutting when m=2. In an original compound 112, two designated locations 114 and 116 are cut. With this process, three compounds 112A, 112B, and 112C are generated from the original compound 112. While all combinations of two designated locations in the original compound 112 are sequentially selected, the cutting of the original compound 112 is repeated.

Referring to FIG. 7, the plurality of derived compounds generated by the cutting process are registered in the memory 92A (S16). A group of primary compounds 94 is formed from all compounds registered in the memory 92A. Then, a bond changing process is applied to compounds which are sequentially selected from the group of primary compounds 94 (S18). Specifically, presuming k=8, while numbers 1 and 2 are selected as a change location number n, a bond changing is repeatedly performed on the selected primary compound as follows. With this process, one or a plurality of derived compounds are generated from one primary compound. In the group of primary compounds 94, primary compounds which do not satisfy the application condition are removed from the target of the bond changing process.

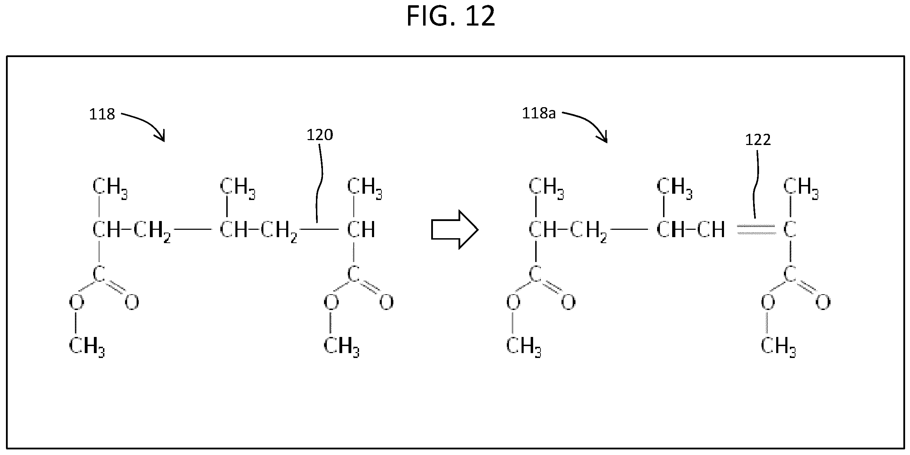

FIG. 12 shows an example of the bond changing when n=1. In a primary compound 118, a designated single bond 120 is changed to an unsaturated bond 122, so that a different compound 118a is generated. In the bond changing, a chemical condition, more specifically, the valence, is taken into consideration. If another single bond can be changed to the unsaturated bond in the primary compound 118, such bond changing is also performed. A similar process is performed also in the case of n=2.

Referring to FIG. 7, a plurality of derived compounds generated by the bond changing process are registered in a memory 92B (S20). In addition, the group of primary compounds 94 is also registered in the memory 92B (S22). A group of secondary compounds 96 is formed by all compounds registered in the memory 92B. The group of secondary compounds 96 is a group of pyrolysis products (a group of compounds) corresponding to a particular resin candidate.

The above-described processes are repeatedly performed for each resin candidate. With this process, a plurality of groups of pyrolysis products corresponding to the plurality of resin candidates are obtained. As described, the actual substance of each individual pyrolysis product is the structural formula. The above-described cutting process is a process for producing a plurality of new derived structural formulae. The above-described bond changing process is also a process for producing a plurality of new derived structural formulae.

The entirety of the sequence of processes shown in FIG. 7 is basically executed by the processor. Alternatively, a portion of the processes may be executed manually. For each resin candidate, for example, 100 to 1,000,000 pyrolysis products are generated. In the case where a pyrolysis product is newly generated which is identical to the pyrolysis product which is already generated during the generation of the group of pyrolysis products for each resin candidate, the newly generated pyrolysis product is discarded.

As already described, for each resin candidate, a plurality of structural formulae representing the plurality of pyrolysis products are sequentially supplied to the prediction model (refer to FIG. 3). With this process, a plurality of predicted mass spectra are sequentially generated. As a result, a group of predicted mass spectra is generated for each resin candidate.

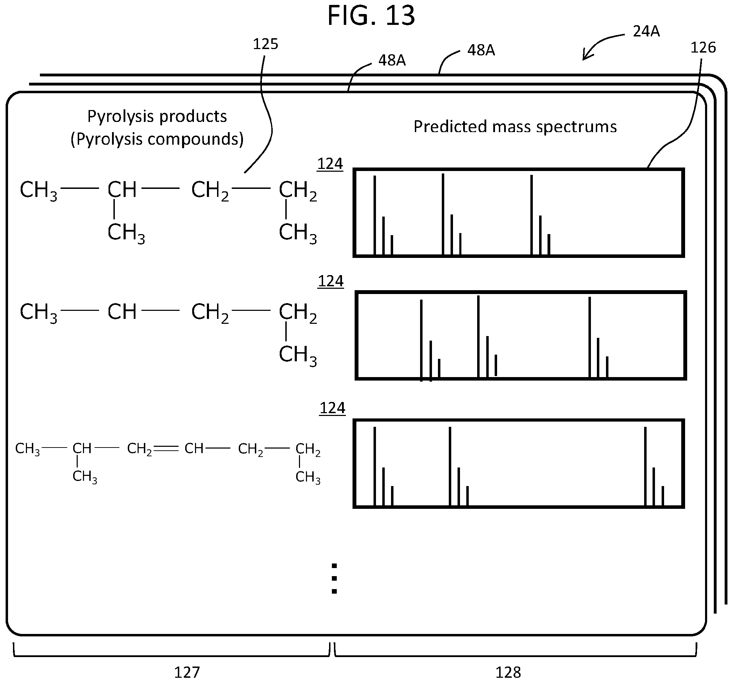

FIG. 13 shows an example of the pyrolysis product library. A pyrolysis product library 24A is formed from a plurality of sub-libraries 48A corresponding to the plurality of resin candidates. Each sub-library 48A is formed from a plurality of records 124. Each record 124 is formed from a structural formula 125 and a mass spectrum 126. From a different perspective, each sub-library 48A is formed from a group of structural formulae 127 and a group of predicted mass spectra 128.

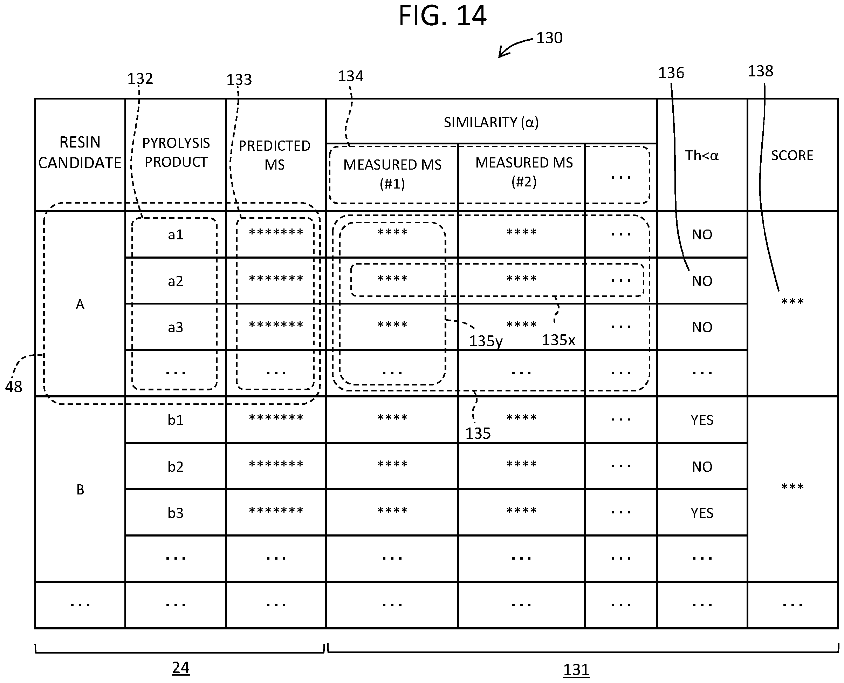

FIG. 14 shows an example of the management table. A management table 130 illustrated in FIG. 14 is an operation table including the pyrolysis product library 24. Alternatively, the pyrolysis product library 24 and a recording area 131 for operation may be set as separate structures.

As already described, the pyrolysis product library 24 is formed from a plurality of sub-libraries 48 corresponding to the plurality of resin candidates. Each sub-library 48 has a group of pyrolysis products (in reality, group of structural formulae) 132 and a group of predicted mass spectra 133. In FIG. 14, each individual predicted mass spectrum is abstractly represented. The group of predicted mass spectra 133 is formed from j predicted mass spectra. Here, j is an integer greater than or equal to 2. For example, j is within a range of 100 to 1,000,000. In general, the value of j differs depending on the resin candidate.

A measured mass spectrum set 134 is formed from i measured mass spectra corresponding to i compounds generated due to pyrolysis of the resin sample. In FIG. 14, a label (#1) is attached to a first measured mass spectrum, and a label (#2) is attached to a second measured mass spectrum. The number i is an integer greater than or equal to 2, and is, for example, in a range of 10 to 50. The numerical values described in the present disclosure are merely exemplary.

For each resin candidate, mass spectrum matching is performed between the measured mass spectrum set 134 and the group of predicted mass spectra 133. Specifically, for each measured mass spectrum, the measured mass spectrum is compared with the group of predicted mass spectrum 133 of each resin candidate, and j similarities α are thus calculated. This process is applied for each of the i predicted mass spectra. As a result, a similarity matrix 135 is obtained. The similarity matrix 135 is formed from (i x j) similarities α. Reference numeral 135y shows a similarity column formed from j similarities α, and reference numeral 135x shows a similarity row formed from i similarities α.

In the embodiment, for each similarity matrix 135, a number of similarities α exceeding the threshold th is counted. The count is used as the score.

More specifically, for each pyrolysis product, it is checked whether or not there is a similarity α exceeding the threshold th in the similarity row 135x. When the maximum value of the similarity is, for example, 1000, the threshold th is, for example, 700. For each similarity row 135x, if there is a similarity α exceeding the threshold th, YES is registered as a flag, and, if there is no such similarity α, NO is registered as the flag (refer to reference numeral 136). By counting the number of YES's, a score 138 is determined. This process is repeatedly performed for each resin candidate. The score 138 is in brief a hit percentage, and more specifically shows the magnitude of the probability that the resin candidate is contained in the resin sample. In the embodiment, the score 138 is a count value.

Alternatively, for each measured mass spectrum, it may be checked whether or not there is a similarity α exceeding the threshold th in the similarity row 135y. For example, when there is no similarity α exceeding the threshold th, a second attribute (other than pyrolysis product) may be judged as the attribute of the compound corresponding to the measured mass spectrum.

Of the plurality of scores corresponding to the plurality of resin candidates, a superior score exceeding a judgment value is identified, and the resin candidate corresponding to the superior score is judged as a resin contained in the resin sample (that is, the contained resin). The judgment value is, for example, 10. When a plurality of superior scores are identified, a plurality of contained resins are judged. The judgment value may be common over the plurality of resin candidates, or changed for each individual resin candidate. For example, the judgment value may be adaptively set based on the value of j for each individual resin candidate. Alternatively, a resin candidate corresponding to the best score may be judged as the contained resin. Alternatively, as a result of the judgment, a score list after being sorted in a descending order may be displayed along with the resin candidate list.

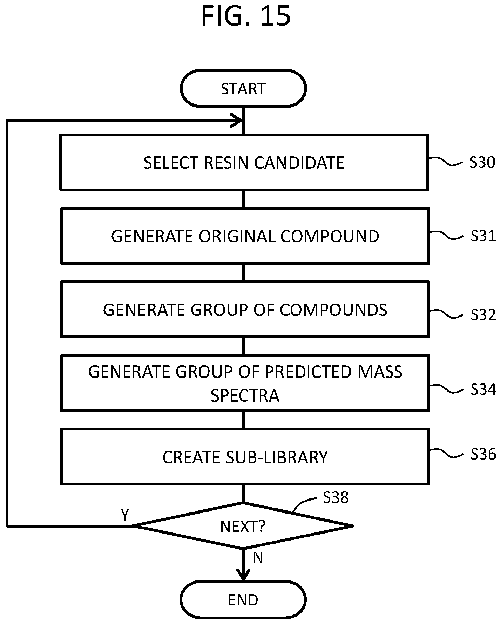

FIG. 15 shows a method of creating the pyrolysis product library as a flowchart. In S30, one resin candidate is selected from the resin candidate list. In S31, an original compound (more specifically, the structural formula) is generated based on the selected resin candidate. Alternatively, in S31, a plurality of original compounds may be generated based on the selected resin candidate. In S32, the group of compounds is generated through the cutting process and the bond changing process of the original compound. The group of compounds also includes the original compound. In S34, the group of predicted mass spectra is generated based on the group of compounds. In S36, a sub-library is formed from the group of compounds. In S38, presence or absence of a next resin candidate is judged, and, if there is the next resin candidate, the processes of S30 to S36 described above are applied to the next resin candidate. Through these processes, the pyrolysis product library formed from the plurality of sub-libraries is created.

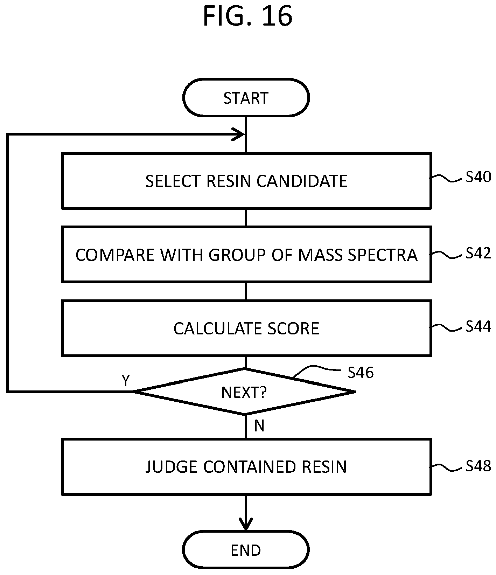

FIG. 16 shows the method of analyzing the resin sample as a flowchart. In S40, one resin candidate is selected from the plurality of resin candidates. In S42, mass spectrum matching is performed between the measured mass spectrum set and the group of predicted mass spectra corresponding to the selected resin candidate. With this process, the similarity matrix is calculated. In S44, the score is calculated based on the similarity matrix. In S46, it is judged whether or not there is a next resin candidate, and, if there is the next resin candidate, the processes from S40 to S44 are applied to the next resin candidate. In S48, one or a plurality of contained resins in the resin sample are judged based on the score array. A result of the judgment is displayed. Alternatively, the comparison between the selected measured mass spectrum and the plurality of groups of mass spectra may be repeatedly performed.

In the resin analyzing system described above, the resin sample is analyzed through individual identification of a plurality of compounds (that is, a plurality of pyrolysis products) generated from the resin sample. Therefore, even if the resin sample contains an impurity, the analysis precision does not tend to be degraded. In addition, a plurality of resins contained in the resin sample can be identified simultaneously. Because retention time information is not used for the resin analysis, the analysis precision can be maintained even when the pyrolysis condition or the component separation condition changes.

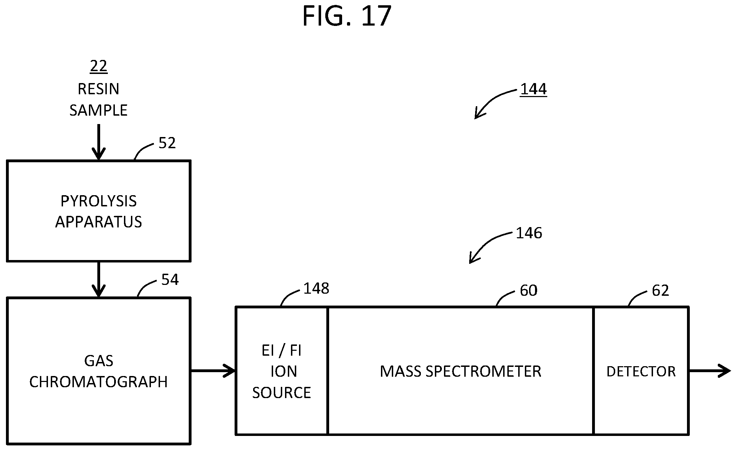

Next, a resin analyzing system according to a second embodiment of the present disclosure will be described with reference to FIGs. 17 to 19. Similar to the resin analyzing system according to the first embodiment of the present disclosure, the resin analyzing system according to the second embodiment comprises a prediction model generation apparatus, a library creation apparatus, and a resin analyzing apparatus (refer to FIG. 1). In the following, parts of the resin analyzing system according to the second embodiment which differ from the resin analyzing system according to the first embodiment will be described.

FIG. 17 shows a structure of a measurement unit 144 according to the second embodiment of the present disclosure. In FIG. 17, constituent elements identical to the constituent elements shown in FIG. 4 are assigned the same reference numerals, and repeated description thereof will be omitted.

In the measurement unit 144, a mass spectrometer 146 has a composite ion source 148. The composite ion source 148 includes an EI ion source which follows the electron ionization method (EI method) and an FI ion source which follows a chemical ionization method (FI method). These ion sources are selectively used. The EI method is one of the hard ionization methods, and the FI method is one of the soft ionization methods. When the hard ionization method is employed, fragment ions tend to be more easily observed. When the soft ionization method is employed, the molecular ions tend to be more easily observed. Alternatively, a hard ionization method other than the EI method may be employed, or a soft ionization method other than the FI method may be employed.

In the second embodiment, a first measurement on a resin sample, and a second measurement on the same resin sample are sequentially performed. For example, during the first measurement, the EI ion source is used, and first detection data is output from the mass spectrometer 146. During the second measurement, the FI ion source is used, and second detection data is output from the mass spectrometer 146.

The generation unit in the information processing unit according to the second embodiment generates a first mass spectrum array based on the first detection data, and generates a second mass spectrum array based on the second detection data. Then, the generation unit generates a first TICC (first pyrogram) based on the first mass spectrum array, and generates a second TICC (second pyrogram) based on the second mass spectrum array.

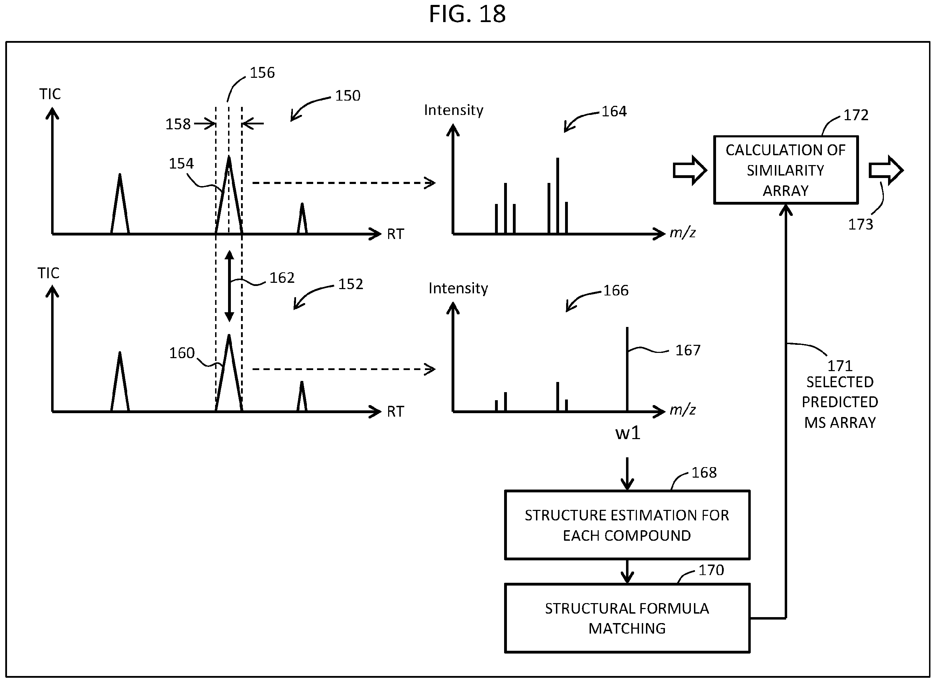

FIG. 18 schematically shows processes executed by the generation unit and the analyzing unit in the information processing unit. In a first TICC 150 and a second TICC 152, the horizontal axis shows the retention time (RT), and the vertical axis shows the total ion current (TIC).

The retention time axis of the first TICC 150 and the retention time axis of the second TICC 152 are correlated with each other, and are parallel to each other in FIG. 18. The first TICC 150 includes a plurality of compound peaks (first compound peak array). Similarly, the second TICC 152 includes a plurality of compound peaks (second compound peak array).

The generation unit repeatedly performs peak pairing between the first compound peak array and the second compound peak array, and, based on the result of the peak pairing, generates a first measured mass spectrum array based on the first mass spectrum array and generates a second measured mass spectrum array based on the second mass spectrum array.

More specifically, for example, a peak search range 158 is determined based on a representative position (for example, a vertex position, or a center-of-gravity position) of the first compound peak 154, and the second TICC 152 is searched through, for a second compound peak 160 belonging to the peak search range 158. With this process, the first compound peak 154 and the second compound peak 160 are correlated with each other. This peak pairing process is sequentially performed along the retention time axis.

For each first compound peak, an accumulation segment is determined, and a plurality of first mass spectra belonging to the accumulation segment are accumulated. With this process, for each first compound peak, a first accumulated mass spectrum; that is, a first measured mass spectrum, is generated. FIG. 18 shows a first measured mass spectrum 164 corresponding to the first compound peak 154.

Similar to the above process, an accumulation segment is determined for each second compound peak, and a plurality of second mass spectra belonging to the accumulation segment are accumulated. With this process, for each second compound peak, a second accumulated mass spectrum; that is, a second measured mass spectrum, is generated. FIG. 18 shows a second measured mass spectrum 166 corresponding to the second compound peak 160. The first measured mass spectrum 164 and the second measured mass spectrum 166 correspond to each other. That is, these mass spectra are mass spectra representing the same compound.

The first measured mass spectrum 164 includes a plurality of fragment peaks, but does not include a molecular ion peak. The second measured mass spectrum 166 includes a molecular ion peak 167, and additionally includes a small number of fragment ion peaks.

As shown in FIG. 18, the analyzing unit identifies, for each compound, an accurate mass (more specifically, a mass-to-charge ratio) w1 of the compound based on the molecular ion peak 167, and then estimates the structural formula of the compound based on the accurate mass w1 (refer to reference numeral 168). In this process, for example, the types and numbers of elements forming the structural formula are searched through, so that a total sum of the mass based on the structural formula matches the accurate mass within a certain error range. The accurate mass is molecular mass information. Alternatively, other molecular mass information (for example, integral mass or molecular weight) may be used in place of the accurate mass. Alternatively, in identifying the molecular ion peak 167, the first measured mass spectrum 164 and the second measured mass spectrum 166 may be compared with each other.

Next, presuming that a plurality of groups of structural formulae corresponding to a plurality of groups of pyrolysis products of a plurality of resin candidates are registered, the analyzing unit performs structural formula matching between the estimated structural formula and the plurality of registered structural formulae, for each compound. For each compound, the analyzing unit identifies each registered structural formula which is judged as a match in the structural formula matching, and extracts a predicted mass spectrum corresponding to the identified registered structural formula (refer to reference numeral 170). With this process, for each compound, one or a plurality of selected predicted mass spectra to be used for the mass spectrum matching are extracted from among the plurality of groups of predicted mass spectra. A selected predicted mass spectrum array 171 is formed by the one or the plurality of the selected predicted mass spectra. Then, the analyzing unit performs, for each compound, mass spectrum matching between the measured mass spectrum and the selected predicted mass spectrum array 171, to calculate a similarity array (refer to reference numeral 172). Based on a plurality of similarity arrays corresponding to a plurality of compounds, a plurality of similarity matrices are formed corresponding to the plurality of resin candidates (refer to reference numeral 173). The analyzing unit calculates a plurality of scores based on the plurality of similarity matrices, and judges one or a plurality of contained resins based on the plurality of scores.

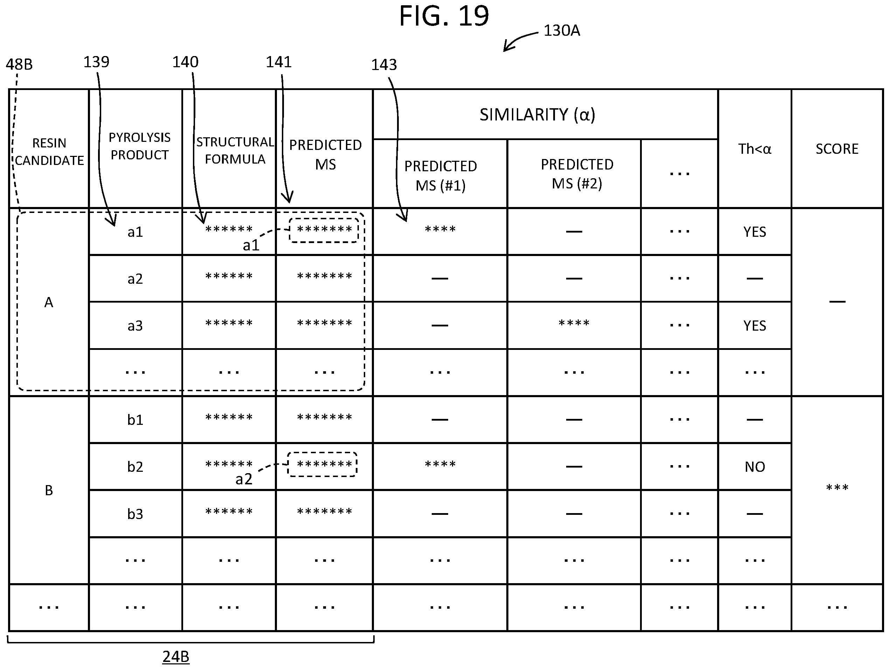

FIG. 19 shows a management table 130A according to the second embodiment of the present disclosure. Reference numeral 24B shows a pyrolysis product library. The pyrolysis product library 24B is formed from a plurality of sub-libraries 48B corresponding to a plurality of resin candidates. Each sub-library 48B has a group of identifiers 139 indicating a group of pyrolysis products, a group of structural formulae (a group of registered structural formulae) 140, and a group of predicted mass spectra 141. As described above, for each compound generated due to the pyrolysis, the structural formula which is estimated (estimated structural formula) is compared with a plurality of groups of registered structural formulae corresponding to the plurality of resin candidates, and one or a plurality of registered structural formulae matching the estimated structural formula are identified. From the plurality of groups of predicted mass spectra 141, one or a plurality of selected predicted mass spectra a1, a2, ... (that is, selected predicted mass spectrum array) corresponding to the one or the plurality of registered structural formulae matching the estimated structural formula are extracted. Reference numeral 143 shows a similarity array calculated between the measured mass spectrum (#1) and the selected predicted mass spectrum array.

According to the second embodiment of the present disclosure described above, because primary judgment based on the molecular mass information and secondary judgment based on the mass spectrum matching are performed stepwise, precision of sample analysis can be improved. Alternatively, in the second embodiment, molecular mass information matching may be performed in place of the structural formula matching. In this case, in the management table 130A shown in FIG. 19, a plurality of molecular mass information may be managed in place of the plurality of structural formulae.

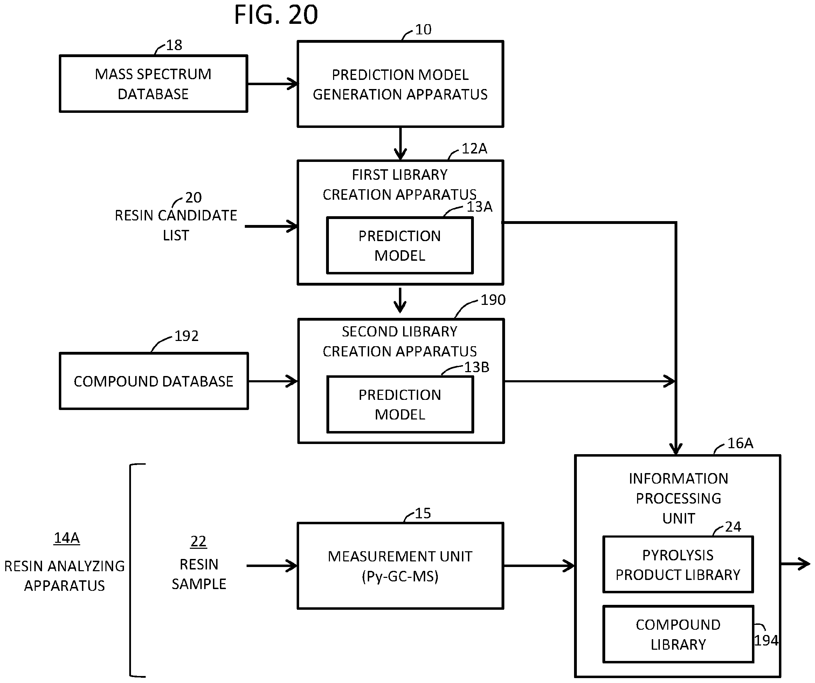

Next, a sample analyzing system according to a third embodiment of the present disclosure will be described with reference to FIGs. 20 to 23. Similar to the resin analyzing system according to the first embodiment of the present disclosure, the resin analyzing system according to the third embodiment comprises a prediction model generation apparatus, a library creation apparatus, and a resin analyzing apparatus (refer to FIG. 1). In the following, parts of the resin analyzing system according to the third embodiment which differ from the resin analyzing system according to the first embodiment will be described.

FIG. 20 shows the resin analyzing system according to the third embodiment of the present disclosure. In FIG. 20, constituent elements identical to the constituent elements shown in FIG. 1 are assigned the same reference numerals, and repeated description thereof will be omitted. In FIG. 20, the resin analyzing system comprises a first library creation apparatus 12A and a second library creation apparatus 190. The first library creation apparatus 12A corresponds to the library creation apparatus according to the first embodiment, and has a prediction model 13A. The prediction model 13A generates a group of predicted mass spectra for each resin candidate in the resin candidate list 20.

The second library creation apparatus 190 has a prediction model 13B which is identical to the prediction model 13A described above. A compound database 192 is an existing compound database, in which a large number of compounds are registered along with the structural formulae thereof. The compound database 192 is, for example, PubChem. The second library creation apparatus 190 has the prediction model 13B similar to the first library creation apparatus, and generates a large number of predicted mass spectra from structural formulae of a large number of compounds. A compound library is formed from these predicted mass spectra. The compound library includes, for example, 100 million or more predicted mass spectra.

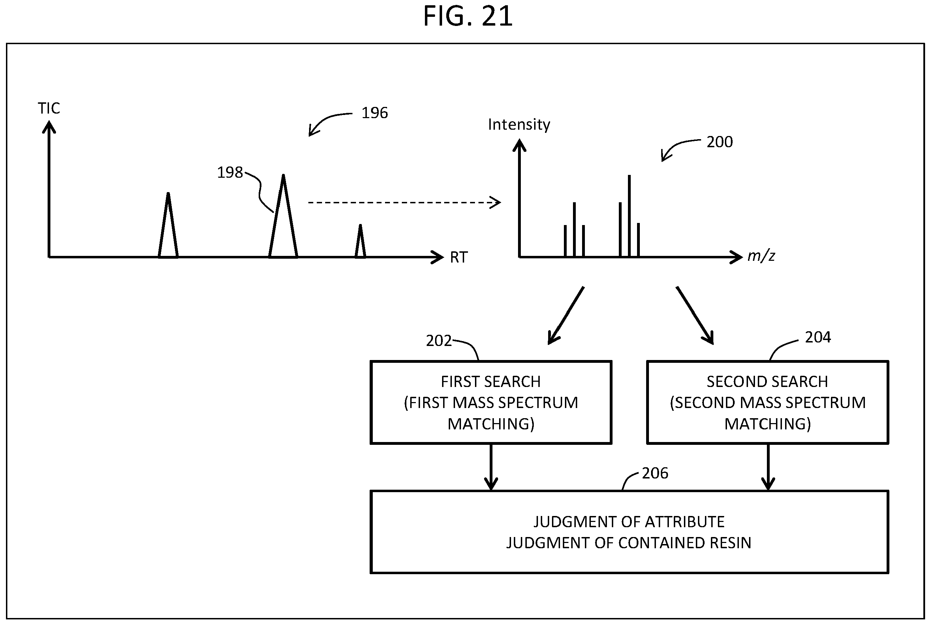

An information processing unit 16A of a resin analyzing apparatus 14A stores the pyrolysis product library 24, and also stores a compound library 194 created by the second library creation apparatus 190. Similar to the information processing unit according to the first embodiment, the information processing unit 16A has a generation unit and an analyzing unit. FIG. 21 shows contents of processes at the analyzing unit.

In FIG. 21, a TICC 196 includes a plurality of compound peaks. A measured mass spectrum 200 is an accumulated mass spectrum corresponding to a compound peak 198. The analyzing unit according to the third embodiment has an attribute judgment function to judge, for each compound peak, whether or not the compound peak corresponds to a pyrolysis product of the resin sample.

More specifically, the analyzing unit searches, for each measured mass spectrum, across the pyrolysis product library and the compound library based on the measured mass spectrum. In FIG. 21, a first search 202 shows a search of the pyrolysis product library, and a second search 204 shows a search of the compound library. Then, as shown by reference numeral 206, the analyzing unit identifies the highest similarity among the result of the first search 202 and the result of the second search. The analyzing unit judges a first attribute (pyrolysis product) as the attribute when the highest similarity results from any of the predicted mass spectra in the pyrolysis product library. On the other hand, when the highest similarity is resulted from any of the predicted mass spectra in the compound library, the analyzing unit judges a second attribute (other compounds) as the attribute. An example of the other compounds is an impurity.

As described, in the third embodiment, for each compound peak included in the TICC, the attribute of the compound corresponding to the compound peak is judged. In the mass spectrum matching, a measured mass spectrum corresponding to the pyrolysis product is used, and the measured mass spectra corresponding to the other compounds are removed. The result of attribute judgment for each compound peak is provided to the user through a reference image to be described below.

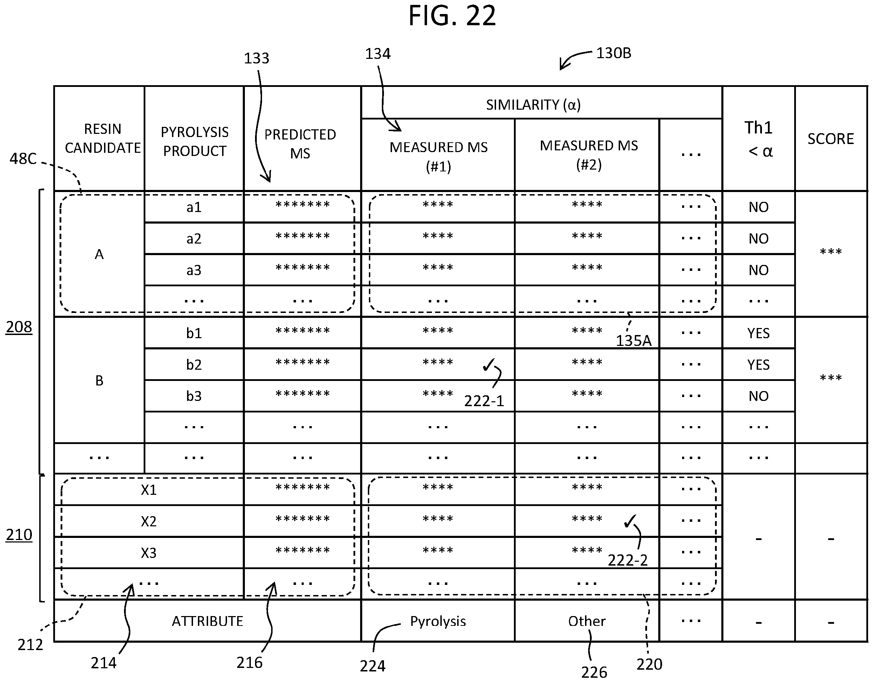

FIG. 22 shows a management table 130B according to the third embodiment of the present disclosure. The management table 130B has a first part 208 and a second part 210. The first part 208 is a part corresponding to the pyrolysis product library, and the second part 210 is a part corresponding to a compound library 212. The pyrolysis product library is formed from a plurality of sublibraries 48C corresponding to a plurality of resin candidates. For each resin candidate, mass spectrum matching is performed between the measured mass spectrum set 134 and the group of predicted mass spectra 133, and a first similarity matrix 135A is thereby calculated.

The compound library 212 has a compound set 214 and a predicted mass spectrum set 216. The mass spectrum matching is performed between the measured mass spectrum set 134 and the predicted mass spectrum set 216, and a second similarity matrix 220 is thereby calculated.

Next, for each measured mass spectrum, the highest similarity is judged from among the plurality of similarities. For example, with regard to the measured mass spectrum (#1), the highest similarity results with a predicted mass spectrum corresponding to a pyrolysis product b2 (refer to reference numeral 222-1). As a result, the first attribute ("Pyrolysis") 224 is judged as the attribute of the compound. On the other hand, with regard to the measured mass spectrum (#2), the highest similarity results with a predicted mass spectrum corresponding to a compound x2 (refer to reference numeral 222-2). As a result, the second attribute ("Other") 226 is judged as the attribute of the compound.

Measured mass spectra judged as having the second attribute among the plurality of measured mass spectra are removed for the score calculation. Specifically, in the example configuration illustrated in FIG. 22, the similarity array corresponding to the measured mass spectrum (#2) is not referred to in the score calculation. As a result, only the measured mass spectra judged as having the first attribute among the plurality of measured mass spectra are determined as effective measured mass spectra, and only the mass spectrum matching results obtained from the effective measured mass spectra are employed as the targets of the score calculation.

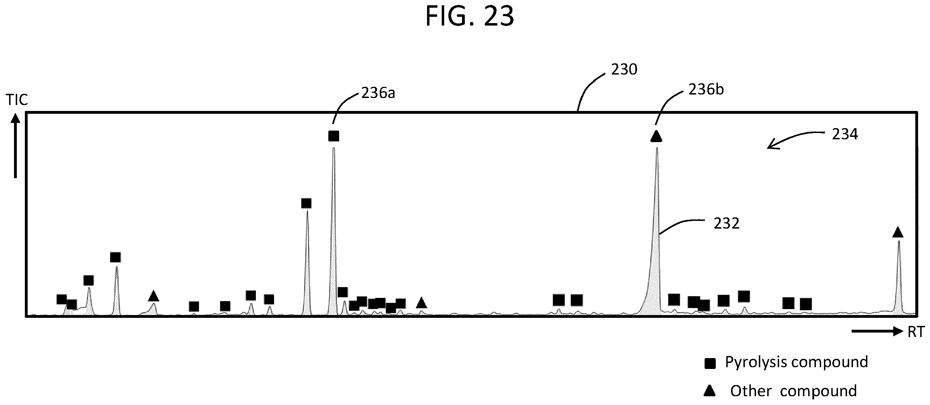

FIG. 23 shows an example display. The display processing unit of the information processing unit has a function to generate a reference image. In FIG. 23, an image 230 has a TICC 232, and a reference image 234 superposed to the TICC 232. The reference image 234 has a plurality of markers corresponding to a plurality of compound peaks. The plurality of markers include a first marker 236a indicating the first attribute (pyrolysis product) and a second marker 236b indicating the second attribute (other compounds). Through observation of the reference image 234, for example, the compound peak may be evaluated, or a content ratio of the impurity can be recognized.

According to the third embodiment described above, among the plurality of measured mass spectra, the effective measured mass spectra may be used for the mass spectrum matching, and, therefore, the analysis precision of the sample can be improved. Desirably, compounds which exist both in the compound library and the pyrolysis product library are removed from the compound library.

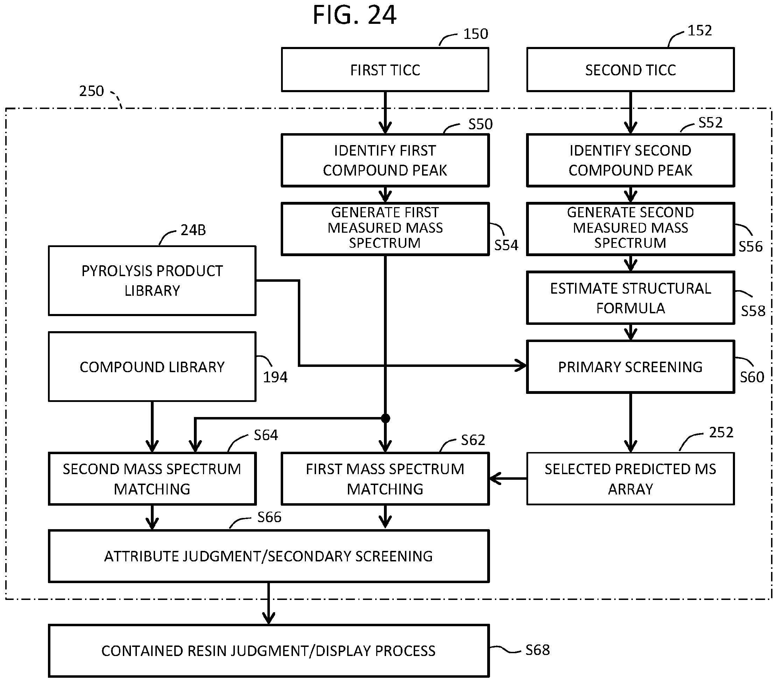

FIG. 24 shows an algorithm executed by a sample analyzing system according to a fourth embodiment of the present disclosure. The fourth embodiment corresponds to a combination of the second embodiment and the third embodiment. Similar to the resin analyzing systems according to the first through third embodiments, the resin analyzing system according to the fourth embodiment comprises a prediction model generation apparatus, a library creation apparatus, and a resin analyzing apparatus. The algorithm is executed by the information processing unit.

In FIG. 24, under the hard ionization method, the first TICC 150 and the second TICC 152 are generated from the resin sample. The first TICC 150 includes a plurality of first compound peaks arranged on the retention time axis, and, similarly, the second TICC 152 includes a plurality of second compound peaks arranged on the retention time axis. For each compound, peak pairing is performed between the plurality of first compound peaks and the plurality of second compound peaks (refer to FIG. 18).

In FIG. 24, a plurality of processes shown in a block 250 are performed for each compound (hereinafter, referred to as a "compound of interest"). In S50 and S52, a first compound peak and a second compound peak corresponding to the compound of interest are identified. In S54, a first measured mass spectrum is generated based on the first compound peak, and, in S56, a second measured mass spectrum is generated based on the second compound peak.

In S58, a molecular ion peak included in the second measured mass spectrum is identified, and, based on a mass-to-charge ratio corresponding to the molecular ion peak, molecular mass information; that is, the accurate mass, of the compound of interest is identified. In S60, a structural formula of the compound of interest is estimated based on the accurate mass.

The pyrolysis product library 24B includes a plurality of predicted mass spectra corresponding to a plurality of pyrolysis products, and a plurality of structural formulae corresponding to the plurality of pyrolysis products. In S60, the estimated structural formula of the compound of interest is compared with a plurality of registered structural formulae in the pyrolysis product library 24B, and a pyrolysis product (selected pyrolysis product) corresponding to each registered structural formula matching the estimated structural formula is identified. Through this primary screening, one or a plurality of selected pyrolysis products are extracted for each compound of interest. That is, one or a plurality of selected predicted mass spectra are extracted. A selected predicted mass spectrum array 252 is formed by the one or the plurality of selected predicted mass spectra.

In S62, first mass spectrum matching is performed between the first measured mass spectrum and the selected predicted mass spectrum array 252, and a similarity is calculated for each selected predicted mass spectrum. In the meantime, in S64, second mass spectrum matching is performed between the first measured mass spectrum and the predicted mass spectrum set in the compound library 194, and a similarity is calculated for each predicted mass spectrum.

In S66, the highest similarity among the plurality of similarities calculated in S62 and the plurality of similarities calculated in S64 is identified. When the highest similarity results from a predicted mass spectrum belonging to the pyrolysis product library 24B, the first attribute (pyrolysis product) is judged as the attribute of the compound of interest, and the highest similarity is assumed to be an effective similarity. That is, the highest similarity is referred to in the score calculation. On the other hand, when the highest similarity results from a predicted mass spectrum belonging to the compound library 194, the second attribute (other compounds) is judged as the attribute of the compound of interest. In this manner, in the fourth embodiment, secondary screening is performed based on the attribute of the compound of interest. The above-described processes are sequentially applied to each of the plurality of compounds generated due to pyrolysis.

In S68, for each resin candidate, a score is calculated based on one or a plurality of effective similarities corresponding to the resin candidate. For a resin candidate for which no effective similarity is calculated, 0 may be assigned as the score. Then, in S68, each of resin candidates having a score exceeding a threshold is judged as the contained resin. In the meantime, in S68, a reference image is generated based on the result of the attribute judgment, and is displayed along with the first TICC (in some cases, along with the first TICC and the second TICC). In the fourth embodiment also, a similarity matrix is generated for each resin candidate, and the score is calculated for each similarity matrix. However, the numbers of effective similarities included in the similarity matrices would differ from each other. In the fourth embodiment, each similarity matrix includes a relatively large number of invalid elements (a similarity of 0 or a blank).

In the fourth embodiment, because the primary screening using the structural formula and the secondary screening using the compound library are performed, the resin analysis precision can be improved. Alternatively, other screening techniques may be further applied with respect to the fourth embodiment.

**Claims**

1. A sample analyzing apparatus comprising:

   a measurement unit (15, 144) that performs mass spectrometry on a plurality of compounds generated due to pyrolysis of a polymer sample;
   a generation unit (65) that generates a measured mass spectrum set, formed from a plurality of measured mass spectra corresponding to the plurality of compounds, based on data which is output from the measurement unit (15, 144); and
   an analyzing unit (66) that analyzes the polymer sample by comparing the measured mass spectrum set with all or a part of a plurality of groups of predicted mass spectra corresponding to a plurality of polymer candidates, wherein
   the group of predicted mass spectra corresponding to each of the polymer candidates includes a plurality of predicted mass spectra corresponding to a plurality of pyrolysis products which may be generated from the polymer candidate.

2. The sample analyzing apparatus according to claim 1, wherein
   each of the predicted mass spectra is a mass spectrum generated by supplying a structural formula representing each of the pyrolysis products to a trained prediction model (13, 32).

3. The sample analyzing apparatus according to claim 1 or 2, wherein
   the analyzing unit (66):

   calculates a score for each of the polymer candidates by performing mass spectrum matching between the measured mass spectrum set and all or a part of the plurality of groups of predicted mass spectra; and
   judges one or a plurality of polymers contained in the polymer sample based on the score for each of the polymer candidates.

4. The sample analyzing apparatus according to claim 3, wherein
   the analyzing unit (66):

   calculates a similarity matrix for each of the polymer candidates as a result of the mass spectrum matching; and
   calculates the score based on the similarity matrix, for each of the polymer candidates.

5. The sample analyzing apparatus according to claim 4, wherein
   the analyzing unit (66):

   counts a number of similarities which are higher than a threshold in the similarity matrix, for each of the polymer candidates; and
   calculates the score based on the number of similarities which are higher than the threshold, for each of the polymer candidates.

6. The sample analyzing apparatus according to claim 1, wherein
   the analyzing unit (66) extracts a selected predicted mass spectrum array to be compared with each of the measured mass spectra, from among the plurality of groups of predicted mass spectra, based on molecular mass information of each of the compounds.

7. The sample analyzing apparatus according to claim 6, wherein
   the analyzing unit (66):

   estimates, based on the molecular mass information of each of the compounds, a structural formula of the compound; and
   extracts the selected predicted mass spectrum array by comparing the structural formula which is estimated with a group of structural formulae corresponding to a group of pyrolysis products of each of the polymer candidates.

8. The sample analyzing apparatus according to claim 6 or 7, wherein

   the measurement unit (144) has a first ion source (148) and a second ion source (148) which differ from each other,
   the generation unit (65):

   generates a first measured mass spectrum set as the measured mass spectrum set based on first data which is output from the measurement unit (144) when the first ion source (148) is used; and

generates a second measured mass spectrum set based on second data which is output from the measurement unit (144) when the second ion source (148) is used, and the analyzing unit (66) identifies a plurality of molecular mass information of the plurality of compounds based on a plurality of molecular ion peaks included in the second measured mass spectrum set.

9. The sample analyzing apparatus according to claim 1, further comprising:

a pyrolysis product library (24) having the plurality of groups of predicted mass spectra; and a compound library (194) having a mass spectrum set corresponding to a known compound set, wherein

the analyzing unit (66):

searches through the pyrolysis product library (24) and the compound library (194) based on the measured mass spectrum set; and

judges, for each of the measured mass spectra, an attribute of a compound corresponding to the measured mass spectrum, based on a result of search of the pyrolysis product library (24) and the compound library (194).

10. The sample analyzing apparatus according to claim 9, wherein

the mass spectrum set includes a plurality of predicted mass spectra generated from a plurality of known structural formulae corresponding to a plurality of known compounds.

11. The sample analyzing apparatus according to claim 9, wherein

the analyzing unit (66) judges the attribute of the compound by identifying a library to which a mass spectrum resulting in a highest similarity belongs.

12. The sample analyzing apparatus according to claim 9, further comprising:

a TICC generator (16A) that generates a total ion current chromatogram (TICC) including a plurality of compound peaks corresponding to the plurality of measured mass spectra, based on the data which is output from the measurement unit (144); and

a reference image generator (16A) that generates a reference image to be displayed along with the TICC, based on attributes of a plurality of compounds identified according to the plurality of measured mass spectra, wherein

the reference image includes a plurality of markers to be displayed along with the plurality of compound peaks, and which show the attributes of the plurality of compounds.

13. A non-transitory storage medium storing a program, which, when executed by an information processing apparatus (16), causes the information processing apparatus (16) to realize the functions of:

comparing (66) a measured mass spectrum set, formed from a plurality of measured mass spectra corresponding to a plurality of compounds generated due to pyrolysis of a polymer sample, with all or a part of a plurality of groups of predicted mass spectra corresponding to a plurality of polymer candidates; and

analyzing (66) the polymer sample based on a result of comparison of the measured mass spectrum set with all or a part of the plurality of groups of predicted mass spectra, wherein

the group of predicted mass spectra corresponding to each of the polymer candidates includes a plurality of predicted mass spectra corresponding to a plurality of pyrolysis products which may be generated from the polymer candidate.

14. A method of creating a pyrolysis product library, comprising:

a step (S30-S32) of generating, for each polymer candidate of a plurality of polymer candidates, a plurality of structural formulae representing a plurality of pyrolysis products theoretically derived from the polymer candidate;

a step (S34) of generating, for each of the polymer candidates, a group of predicted mass spectra, formed from a plurality of predicted mass spectra, based on the plurality of structural formulae; and

a step (S34) of creating a pyrolysis product library including a plurality of groups of predicted mass spectra corresponding to the plurality of polymer candidates.

15. The method of creating the pyrolysis product library according to claim 14, wherein

the step (S30-S32) of generating the plurality of structural formulae comprises:

a step of generating an original structural formula of an original pyrolysis product serving as a monomer connecting structure; and

a step of generating a plurality of derived structural formulae from the original structural formula, and

the plurality of structural formulae include the original structural formula and the plurality of derived structural formulae.

# FIG. 1

FIG. 2

10 PREDICTION MODEL GENERATION APPARATUS

MASS SPECTRUM DATABASE 18

CONVERSION UNIT 26

MODEL GENERATION UNIT 30

PREDICTION MODEL (GCN) 32

34

36

38

# FIG. 3

12

24

20
RESIN
CANDIDATE
LIST

40

PYROLYSIS
PRODUCT
GENERATION
UNIT

42

CONVERSION
UNIT

44

PREDICTION
UNIT

13

PREDICTION
MODEL

SUB-LIBRARY

48

20

## FIG. 4

# FIG. 5

16 INFORMATION PROCESSING UNIT

from 15

64

67 MASS SPECTRUM GENERATOR

68 TICC GENERATOR

70 PEAK DETECTOR

72 ACCUMULATOR

65 GENERATION UNIT

76 DISPLAY PROCESSING UNIT

66 ANALYZING UNIT

74 SIMILARITY CALCULATOR

78 SCORE CALCULATOR

80 JUDGMENT UNIT

PYROLYSIS PRODUCT LIBRARY — 24

EP 4 435 793 A2

22

FIG. 6

| Name | Monomer |
|------|---------|
| Polypropylene | |
| Polybutene | |
| Polystylene | |
| Poly(methyl methacrylate) | |
| Poly(ethylene terephthalate) | |
| ⋮ | ⋮ |

FIG. 7

FIG. 8

FIG. 9

FIG. 10

FIG. 11

FIG. 12

# FIG. 13

EP 4 435 793 A2

## FIG. 14

130

| RESIN CANDIDATE | PYROLYSIS PRODUCT | PREDICTED MS | SIMILARITY (α) | | | Th<α | SCORE |
|---|---|---|---|---|---|---|---|
| | | | MEASURED MS (#1) | MEASURED MS (#2) | ... | | |
| A | a1 | ******* | **** | **** | ... | NO | *** |
| | a2 | ******* | **** | **** | ... | NO | |
| | a3 | ******* | **** | **** | ... | NO | |
| | ... | ... | ... | ... | ... | ... | |
| B | b1 | ******* | **** | **** | ... | YES | *** |
| | b2 | ******* | **** | **** | ... | NO | |
| | b3 | ******* | **** | **** | ... | YES | |
| | ... | ... | ... | ... | ... | ... | |
| ... | ... | ... | ... | ... | ... | ... | ... |

132 133 134 135y 135x 135 136 138 48

24     131

31

# FIG. 15

```
         START
           |
           v
  ┌─────────────────────────┐
  │  SELECT RESIN CANDIDATE  │────  S30
  └─────────────────────────┘
           |
           v
  ┌─────────────────────────┐
  │ GENERATE ORIGINAL        │────  S31
  │ COMPOUND                 │
  └─────────────────────────┘
           |
           v
  ┌─────────────────────────┐
  │ GENERATE GROUP OF        │────  S32
  │ COMPOUNDS                │
  └─────────────────────────┘
           |
           v
  ┌─────────────────────────┐
  │ GENERATE GROUP OF        │
  │ PREDICTED MASS           │────  S34
  │ SPECTRA                  │
  └─────────────────────────┘
           |
           v
  ┌─────────────────────────┐
  │   CREATE SUB-LIBRARY     │────  S36
  └─────────────────────────┘
           |
           v        S38
     Y  < NEXT? >
           | N
           v
          END
```

# FIG. 16

START

SELECT RESIN CANDIDATE — S40

COMPARE WITH GROUP OF MASS SPECTRA — S42

CALCULATE SCORE — S44

NEXT? — S46

Y

N

JUDGE CONTAINED RESIN — S48

END

FIG. 17

FIG. 18

## FIG. 19

130A

| RESIN CANDIDATE | PYROLYSIS PRODUCT | STRUCTURAL FORMULA | PREDICTED MS | SIMILARITY (α) | | | Th<α | SCORE |
|---|---|---|---|---|---|---|---|---|
| | | | | PREDICTED MS (#1) | PREDICTED MS (#2) | ... | | |
| A | a1 | ****** a1 | ******* | **** | — | ... | YES | — |
| | a2 | ****** | ******* | — | — | ... | — | |
| | a3 | ****** | ******* | — | **** | ... | YES | |
| | ... | ... | ... | ... | ... | ... | ... | |
| B | b1 | ****** | ******* | — | — | ... | — | *** |
| | b2 | ****** a2 | ******* | **** | — | ... | NO | |
| | b3 | ****** | ******* | — | — | ... | — | |
| | ... | ... | ... | ... | ... | ... | ... | |
| ... | ... | ... | ... | ... | ... | ... | ... | ... |

48B  139  140  141  143

24B

# FIG. 20

MASS SPECTRUM DATABASE ∽18

PREDICTION MODEL GENERATION APPARATUS ∽10

RESIN CANDIDATE LIST ∽20

FIRST LIBRARY CREATION APPARATUS ∽12A

PREDICTION MODEL ∽13A

COMPOUND DATABASE ∽192

SECOND LIBRARY CREATION APPARATUS ∽190

PREDICTION MODEL ∽13B

RESIN ANALYZING APPARATUS 14A

RESIN SAMPLE 22

MEASUREMENT UNIT (Py-GC-MS) ∽15

INFORMATION PROCESSING UNIT ∽16A

PYROLYSIS PRODUCT LIBRARY ∽24

COMPOUND LIBRARY ∽194

# FIG. 21

EP 4 435 793 A2

# FIG. 22

| RESIN CANDIDATE | PYROLYSIS PRODUCT | PREDICTED MS | SIMILARITY ($\alpha$) | | | Th1 $< \alpha$ | SCORE |
|---|---|---|---|---|---|---|---|
| | | | MEASURED MS (#1) | MEASURED MS (#2) | ... | | |
| A | a1 | ****** | **** | **** | ... | NO | *** |
| | a2 | ****** | **** | **** | ... | NO | |
| | a3 | ****** | **** | **** | ... | NO | |
| | ... | ... | ... | ... | ... | ... | |
| B | b1 | ****** | **** | **** | ... | YES | *** |
| | b2 | ****** | **** | **** | ... | YES | |
| | b3 | ****** | **** | **** | ... | NO | |
| | ... | ... | ... | ... | ... | ... | |
| ... | ... | ... | ... | ... | ... | ... | |
| | X1 | ****** | **** | **** | ... | – | – |
| | X2 | ****** | **** | **** | ... | | |
| | X3 | ****** | **** | **** | ... | | |
| | ... | ... | ... | ... | ... | | |
| ATTRIBUTE | | | Pyrolysis | Other | ... | – | – |

FIG. 23

# FIG. 24

FIRST TICC — 150

SECOND TICC — 152

250

S50 — IDENTIFY FIRST COMPOUND PEAK

S52 — IDENTIFY SECOND COMPOUND PEAK

GENERATE FIRST MEASURED MASS SPECTRUM — S54

GENERATE SECOND MEASURED MASS SPECTRUM — S56

24B — PYROLYSIS PRODUCT LIBRARY

ESTIMATE STRUCTURAL FORMULA — S58

COMPOUND LIBRARY — 194

PRIMARY SCREENING — S60

S64 — SECOND MASS SPECTRUM MATCHING

S62 — FIRST MASS SPECTRUM MATCHING

252 — SELECTED PREDICTED MS ARRAY

S66 — ATTRIBUTE JUDGMENT/SECONDARY SCREENING

CONTAINED RESIN JUDGMENT/DISPLAY PROCESS — S68

**REFERENCES CITED IN THE DESCRIPTION**

**Patent documents cited in the description**

- JP 2000035422 A